# EUROPEAN PATENT SPECIFICATION

(11) **EP 0 785 998 B1**
(45) Date of publication and mention of the grant of the patent: **29.07.1998**
(21) Application number: 94930172.5
(22) Date of filing: 14.10.1994
(51) Int. Cl.: C12N 15/11, C12N 9/00, A61K 31/70

(54) **ASYMMETRIC HAMMERHEAD RIBOZYMES AND NUCLEOTIDE SEQUENCES FOR THEIR CONSTRUCTION**
ASYMETRISCHE "HAMMERHEAD" RIBOZYME UND NUKLEOTIDSEQUENZEN ZU DEREN AUFBAU
RIBOZYMES ASYMETRIQUES EN TETE DE MARTEAU ET SEQUENCES DE NUCLEOTIDES DESTINEES A LEUR CONSTRUCTION

(43) Date of publication of application: 30.07.1997
(73) Proprietor: FOUNDATION FOR RESEARCH AND TECHNOLOGY HELLAS (FO.R.T.H.) INSTITUTE OF MOLECULAR BIOLOGY & BIOTECHNOLOGY, Crete (GR); DKFZ DEUTSCHES KREBSFORSCHUNGSZENTRUM STIFTUNG DES ÖFFENTLICHEN RECHTS, 69120 Heidelberg (DE)
(72) Inventor: TABLER, Martin, GR-711 10 Heraklion-Fortetsa (GR); SCZAKIEL, Georg, D-69181 Leimen (DE); HOMANN, Matthias, D-69120 Heidelberg (DE)
(74) Representative: Perrey, Ralf, Dr. Dipl.-Chem.
(86) International application number: EP9403391
(87) International publication number: WO9510609

(56) References cited:
- EP-A- 0 321 201
- WO-A-91/19789
- WO-A-92/01786
- NUCLEIC ACIDS RESEARCH 22 (19). 3958-3965, 25 September 1994 TABLER, M. ET AL. 'A three-nucleotide helix I is sufficient for full activity of a hammerhead ribozyme: Advantages of an asymmetric design.'

## Description

The present invention relates to nucleotide sequences which can be used for construction of target RNA-specific hammerhead ribozymes. The invention also relates to asymmetric hammerhead ribozymes containing said nucleotide sequences, as well as to a process for their preparation. Other embodiments of the present invention will become apparent from the following description.

One class of naturally occurring RNAs which undergo site-specific autolytic cleavage, can assume a so-called "hammerhead" structure (Forster and Symons, Cell **50** (1987), 9-16) determining the site of cleavage. The majority of these self-cleaving RNAs originates from satellite RNAs of plant viruses. There is also one self-cleaving RNA of this type found in another plant pathogen, the avocado sunblotch viroid (ASBVd) and in an RNA transcript of newt (reviewed by Bruening, Method. Enzymol. **180** (1989), 546-558) . A self-catalyzed cleavage reaction has been demonstrated *in vitro* for the avocado sunblotch viroid (ASBVd) (Hutchins et al., Nucleic Acids Res. **14** (1986), 3627-3640), the satellite RNAs of tobacco ring spot virus (sTobRV) (Prody et al., Science **231** (1986), 1577-1580; Buzayan et al., Proc.Natl.Acad.Sci. **83** (1986), 8859-8862), of lucerne transient streak virus (sLTSV) (Forster and Symons, Cell **49** (1987), 211-220) and also for RNA transcripts of repeated DNA sequences from newt (Epstein and Gall, Cell **48** (1987), 535-543).

Using two synthetic RNA oligomers which can together form a hammerhead structure, it was demonstrated that cleavage can occur in trans, i.e. that one molecule can catalyze the cleavage of the other (Uhlenbeck, Nature **328** (1987), 596-600). When cleavage occurs in trans, the RNA which can be cleaved, is considered the substrate (target RNA) and the RNA which catalyzes the cleavage, is designated "ribozyme". Ribozymes assuming a hammerhead structure are designated "hammerhead ribozymes".

It is now possible to design an appropriate ribozyme against any GUC sequence motif, i.e. the cleavable motif, of a given substrate (i.e. the target RNA), so that cleavage occurs 3'-terminal of it (Haseloff and Gerlach, Nature **334** (1988), 585-591). Likewise GUA, GUU, CUC, AUC and UUC are suitable cleavable motifs for the hammerhead ribozymes (Koizumi et al., FEBS Lett. **228** (1988), 228-230 and FEBS Lett. **239** (1988), 285-288, and EP-A2 321 201). The motif GUG was cleaved in one case (Sheldon and Symons, Nucleic Acids Res. **17** (1989), 5679-5685), whereas it was not cleaved in others (Koizumi et al., FEBS Lett. **228** (1988), 228-230, and Haseloff and Gerlach, Nature **334** (1988), 585-591) . In general, any trinucleotide sequence NUH can be a cleavable motif (N can be A, C, G or U; H can be A, C or U) (Ruffner et al., Biochemistry 29, (1990), 10695-10702). Thus, also the motifs AUA, AUU, CUA, CUU, UUA, UUU represent cleavable motifs. It was shown that also CAC, UAC and AAC can be cleaved at a low rate (Perriman et al., Gene **113** (1992), 157-163).

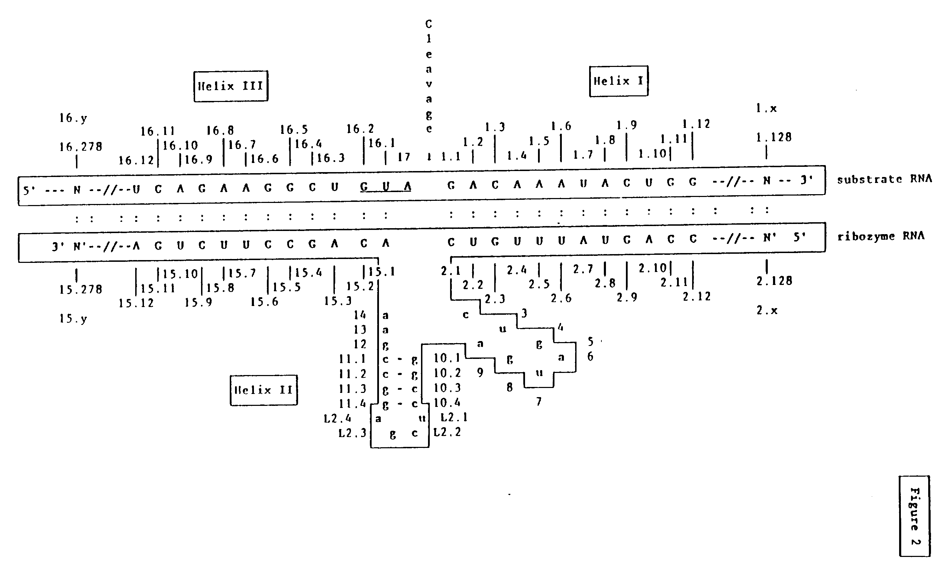

EP-A2 321 201 describes the ribozymes of the "hammerhead"-type and its usage to create RNA endonucleases which are highly specific for a certain target RNA. In order to cleave a given substrate (target RNA) at a certain site, a specific ribozyme RNA can be designed that consists of three functional regions: the actual catalytic domain, and the two regions which are complementary to the target RNA, so that the ribozyme can bind to its substrate in a sequence-specific manner, and in that way that the catalytic domain of the ribozyme is placed opposite of the cleavable motif of the target RNA. The two regions of the ribozyme RNA which are complementary to the target RNA (also called "flanking sequences") can be classified into a region which is located 5' to the catalytic domain and a region located 3' of it. The 5' flanking region consists, according to the numbering system for hammerhead RNAs (Hertel et al., Nucleic Acids Res. **20**, (1992), 3252), of nucleotides 2.1 till 2.x (compare Figure 2), wherein the numbering is from 3' to 5' and x is the most 5' located nucleotide that is base-pairing to the corresponding complementary nucleotides 1.1 till 1.x (numbered from 5' to 3') of the target RNA. The double-stranded region formed between nucleotides 1.1 till 1.x of the target RNA and 2.1 till 2.x of the hammerhead ribozyme-substrate complex is called "Helix I". Similarly, the 3' flanking sequence of the hammerhead ribozyme consists of nucleotides 15.1 till 15.y (numbering 5' to 3') which are able to basepair with the complementary nucleotides 16.1 till 16.y of the target RNA (numbering 3' to 5'). The latter double-stranded region forms "Helix III" of the hammerhead ribozyme-substrate complex. According to this definition the cleavable motif of the substrate RNA is composed of the three nucleotides 16.2, 16.1, 17 (compare Figure 2).
EP-A2 321 201 describes the procedure to create a ribozyme. For this purpose, a DNA oligonucleotide is synthesized according to a specific cleavable motif and the sequence context next to it within the target RNA. After cloning of this DNA oligonucleotide downstream of an appropriate promoter, this synthetic DNA can be used for the *in vitro* and *in vivo* synthesis of the target-specific ribozyme RNA. Transcription generates an RNA molecule which contains the catalytic ribozyme domain, flanked by sequences that are complementary to the target RNA and usually also vector-derived sequences which are not complementary to the target RNA. After the sequence-specific hammerhead conformation has been formed, cleavage occurs 3'-terminal to the cleavable motif of the substrate RNA, e.g. GUC. Since the base-pairing complementary regions between the substrate and the ribozyme RNA, i.e. Helix I and III, which do not participate in the catalytic reaction, enable the sequence-specific binding of the ribozyme to its target, their length influences the specificity and efficiency of the ribozyme reaction.
WO92/01786 describes portable ribozyme cassettes which can be incorporated into certain restriction enzyme cleavage sites of any desired DNA sequence. Transcription into RNA results in catalytic antisense RNAs which are hammerhead RNAs with large Helices I and III. Such catalytic antisense RNAs have also been described elsewhere (Tabler and Tsagris, Gene **108**, (1991), 175-183).

The hammerhead ribozymes known in the art contain two domains that are sequence-specific for the target RNA (Helix I and III) which each are at least 5 nucleotides in length and which are separated by the catalytic domain. Both domains have to be specifically synthesized for each sequence context of a particular cleavable motif in a target RNA or ribozymes have to be generated by inserting particular DNA cassettes into cDNA encoding the target RNA. The latter technique has the disadvantage that ribozyme construction is limited to the availability of certain restriction recognition sequences.
Regardless of their mode of construction, ribozymes known in the art may exhibit low binding rates to their corresponding target RNA and/or low catalytic activity based on thermodynamically or kinetically unfavorable properties.

Thus, the technical problem underlying the present invention is to provide a novel system permitting the target RNA-specific construction of hammerhead ribozymes having high catalytic activity.

The solution to the above technical problem is achieved by providing nucleotide sequences which form, or are able to form, part of a hammerhead ribozyme. Furthermore, it is achieved by providing the other embodiments characterized in the claims.

The present invention relates to a nucleotide sequence comprising the following general formula which is given in 3' - 5' direction in accordance with the antisense polarity of the ribozyme RNA

3'-[N]₁₁-[N]_{Lo}-[N]₁₀-[N]₉₋₃-[N]-5'

wherein N represents any nucleotide and may be the same or different, [N] represents no nucleotide, nucleotide 2.1 or nucleotides 2.x till 2.1 capable of forming Helix I in a hammerhead ribozyme/target-complex, [N]₉₋₃ represents nucleotides 3 to 9 of a hammerhead ribozyme, [N]₁₀ represents nucleotides 10.1 till 10.n and [N]₁₁ represents no nucleotide, nucleotide 11.m or comprises at least a part of nucleotides 11.m till 11.1, x is an integer ranging from 2 to 8, m is an integer, n is an integer and represents the length of Helix II, wherein, if [N]₁₁ comprises at least a part of nucleotides 11.m till 11.1, n and m may be the same or different, Lo is an integer and [N]_{Lo} represents any sequence capable of forming Loop 2 of a hammerhead ribozyme.

The terms "nucleotides 2.x till 2.1", "nucleotide 2.1", "nucleotides 10.1 till 10.n", "nucleotides 11.m till 11.1", "nucleotide 11.m", "Helix I", "Helix II", "nucleotides 3 to 9" and "Loop 2" correspond to the nomenclature and numbering system for hammerhead ribozymes (Hertel et al., supra).
The term "nucleotide sequence" refers to either an RNA sequence contained in a ribozyme RNA or to a DNA sequence contained in a longer DNA sequence or to heterooligomeric sequences.
The term "capable of forming Helix I in a hammerhead ribozyme/target-complex" refers to the region of the ribozyme sequence that flanks the catalytic domain at the 5' side (5' flanking region) and that is complementary to that region of the target RNA that is located 3' of the cleavable motif of the target RNA, so that it can form a double-stranded RNA helix, said helix being Helix I. If the Helix I forming region is contained in a DNA sequence it will be called also "Helix I Box".
The term "capable of forming at least part of the Helix II of a hammerhead ribozyme" refers to the intramolecular double-stranded region found within the catalytic domain of a ribozyme RNA. The integers n and m are starting with 2. In a preferred embodiment of the present invention the integer n ranges from 3 to 50.
The term "any sequence capable of forming Loop 2 of a hammerhead ribozyme" refers to nucleotide sequences which connect the two sequences 10.1 till 10.n with 11.m till 11.1 which form Helix II of a hammerhead ribozyme.
Examples of such a sequence are shown in Figures 1-5, 7-14, 16 and 17, particularly Figure 9 and 17.

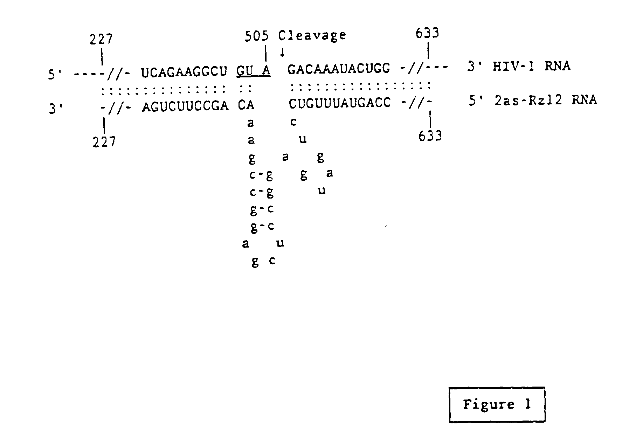

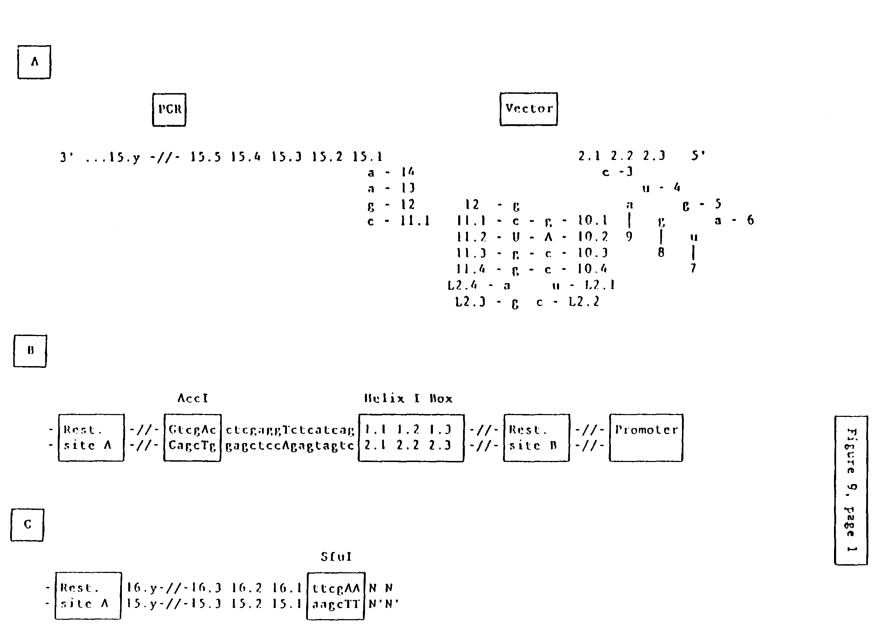

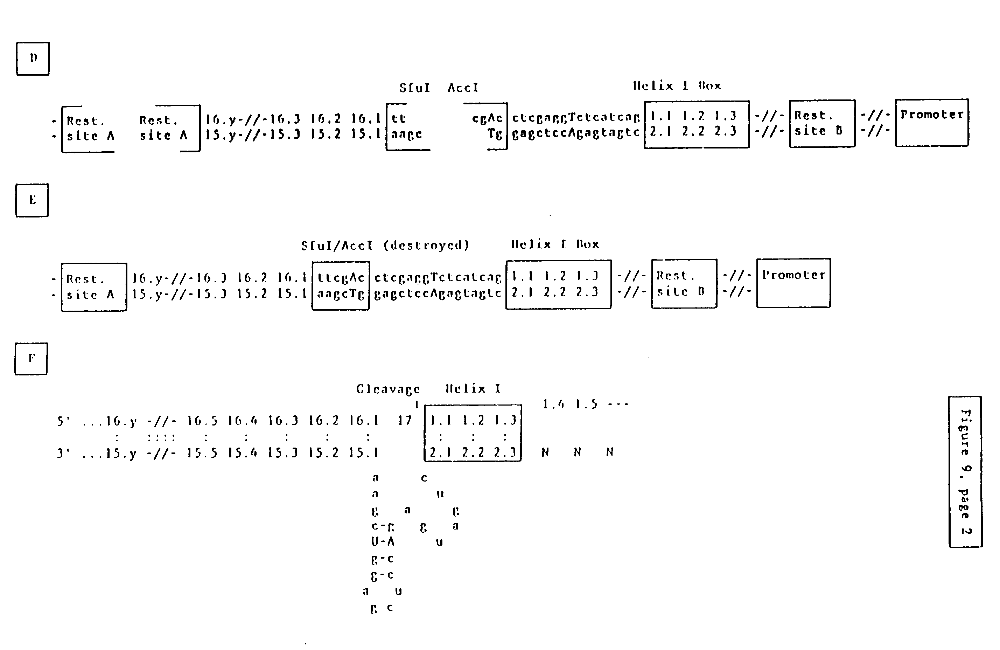

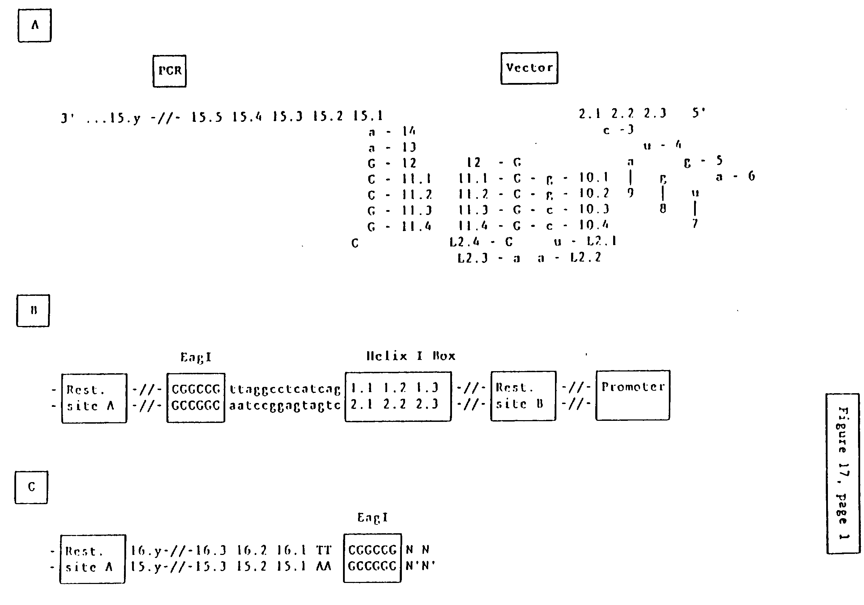

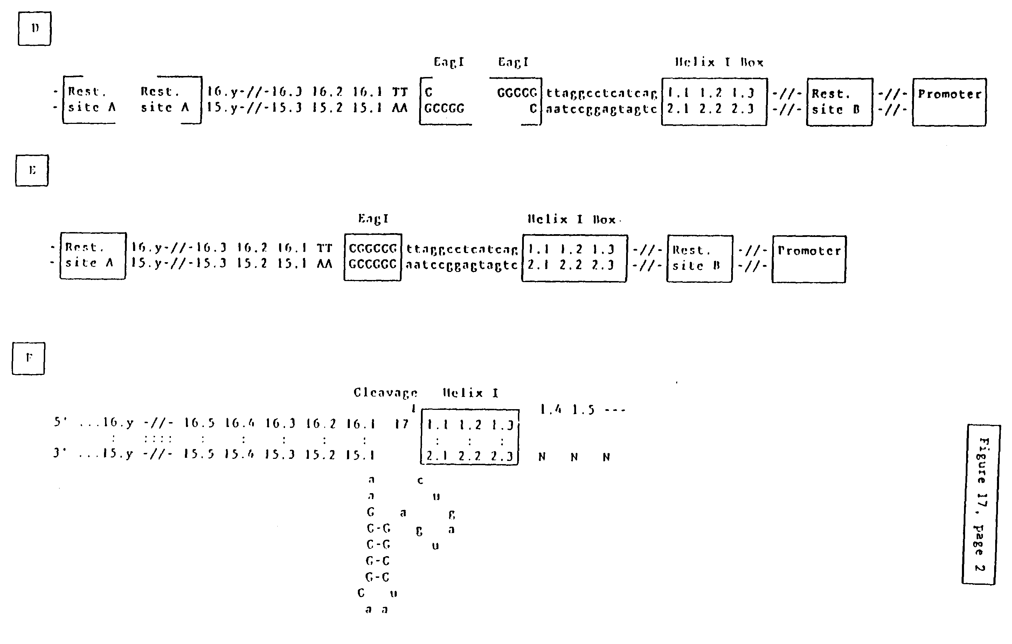

In a preferred embodiment of the present invention the region 3'-[N]₁₁-[N]_{Lo}-[N]₁₀-5' contains at least one recognition sequence for one or more restriction enzymes and/or a recognition sequence for a restriction enzyme that overlaps with the sequence following 3' to [N]₁₁ (compare Figure 9 and 17).
The term "recognition sequence for one or more restriction enzymes" refers to a nucleotide sequence which is cleavable by one or more types of a restriction endonuclease when present in a double-stranded DNA molecule. Examples of such a recognition sequence are shown in Figures 1-5, 7-14, 16 and 17.

Specific examples of the nucleotide sequence of the present invention are listed in the following Table I:

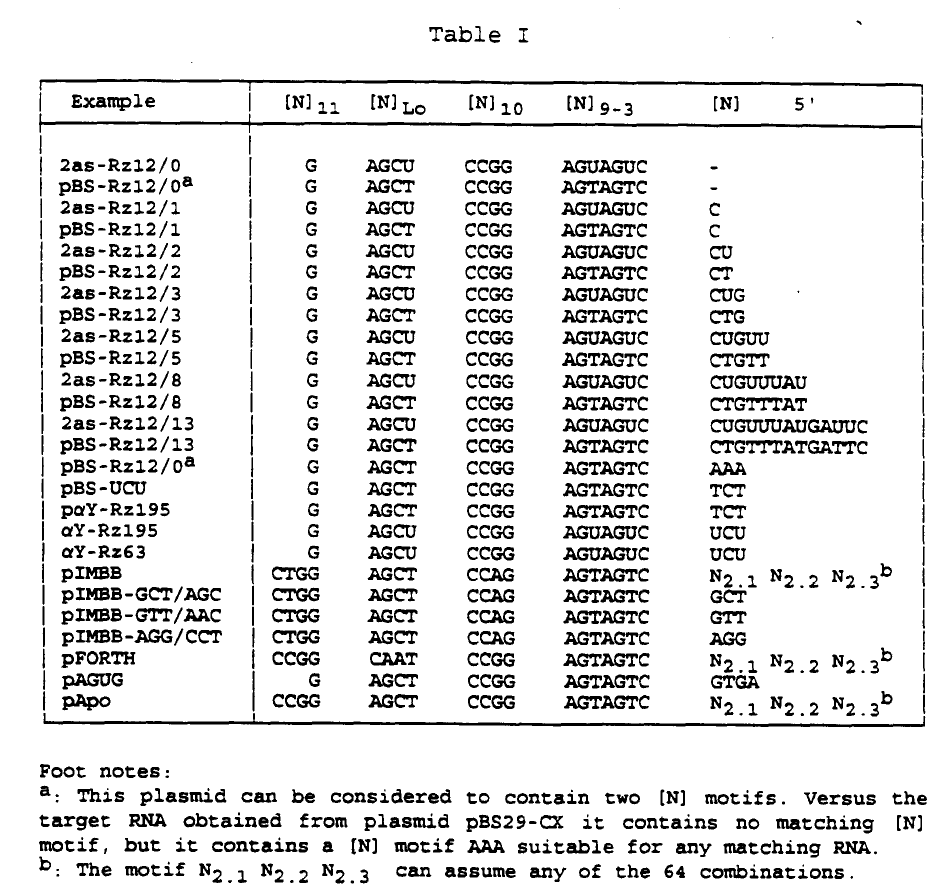

Another embodiment of the present invention relates to a nucleic acid comprising the above defined nucleotide sequence and a second nucleotide sequence containing at least the nucleotides 12 to 14 ("part of the catalytic domain") of a hammerhead ribozyme and at least part of the Helix III-forming region ("Helix III-forming part") of a hammerhead ribozyme/target-complex, starting with nucleotide 15.1 till 15.y, wherein y is an integer.
The terms "nucleotides 12 to 14" and "Helix III" correspond to the nomenclature and numbering system for hammerhead ribozymes (Hertel et al., supra).
The term "nucleic acid" refers to a nucleic acid sequence composed essentially of DNA and/or RNA.
The term "second nucleotide sequence" refers to a nucleotide sequence as defined above which is linked 3' to the above defined nucleotide sequence.
The term "containing at least part of the Helix III-forming region of a hammerhead ribozyme/target-complex" refers to the region of the ribozyme sequence that flanks the catalytic domain at the 3' side (3' flanking region) starting with nucleotide 15.1 of the ribozyme RNA which is complementary to the nucleotide 16.1 in the target RNA which is itself the middle nucleotide of the cleavable motif, consisting of the three nucleotides 16.2, 16.1, and 17, so that it can form a double-stranded RNA helix, said helix being Helix III.
In a preferred embodiment of the present invention the nucleotide sequence and the second nucleotide sequence are linked via one of the recognition sequences contained in 3'-[N]₁₁-[N]_{Lo}-[N]₁₀ 5'or via a recognition sequence for a restriction enzyme that overlaps with the sequence following 3' to [N]₁₁.

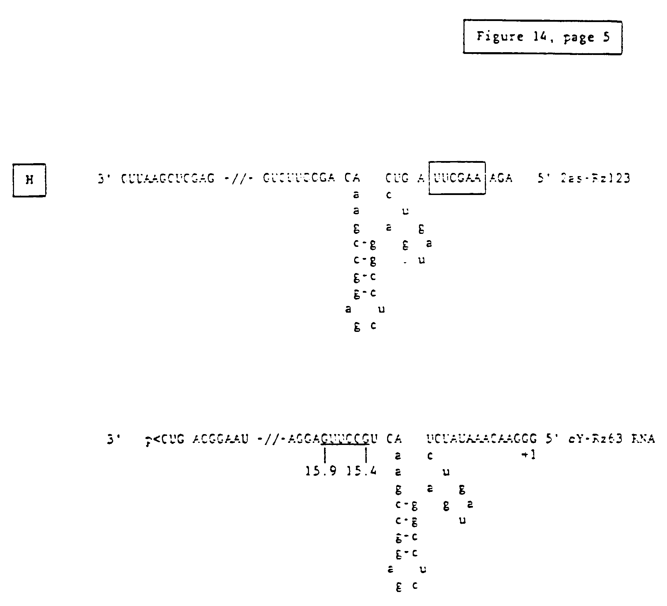

In a further preferred embodiment of the present invention the RNA contains a third nucleotide sequence adjacent to the Helix III-forming region of the second nucleotide sequence, containing at least 3 nucleotides capable of forming an intramolecular Helix III of a hammerhead ribozyme, followed directly by nucleotide 17 of a hammerhead ribozyme and a nucleotide sequence which is complementary to [N].
The term "nucleotide 17" corresponds to the nomenclature and numbering system for hammerhead ribozymes (Hertel et al., supra).
The term "third nucleotide sequence adjacent to the Helix III-forming region of the second nucleotide sequence" refers to a nucleotide sequence which is located 3' to the above defined second nucleotide sequence and it can be either linked directly or intervened by an unrelated sequence.
The term "containing at least 3 nucleotides capable of forming an intramolecular Helix III of a hammerhead ribozyme" refers to nucleotides which correspond to nucleotides 16.3, 16.2, 16.1 in the target RNA.
The term "nucleotide sequence which is complementary to [N]" refers to nucleotides which would correspond to nucleotides 1.1 and following nucleotides in the target RNA and which are complementary to the above defined sequence [N].
A specific example of such an RNA is shown in Figure 14.

Another embodiment of the present invention relates to an asymmetric hammerhead ribozyme comprising the above defined RNA or nucleotide sequence, wherein the Helix I-forming region is smaller than the Helix III-forming region.
The term "the Helix I-forming region is smaller than the Helix III-forming region" refers to different number of nucleotides of the ribozyme sequence that are complementary to the target RNA and that are able to form Helix I and Helix III, wherein the number of nucleotides within the Helix I-forming part, corresponding to nucleotides 2.1 till 2.x in the general formula, is smaller than the number of the nucleotides of the Helix III-forming region, corresponding to nucleotides 15.1 till 15.y in hammerhead ribozymes. The integer x ranges preferably from 2 to 8. In view of the different length of the two flanking sequences these type of ribozymes will be called "asymmetric hammerhead ribozymes".

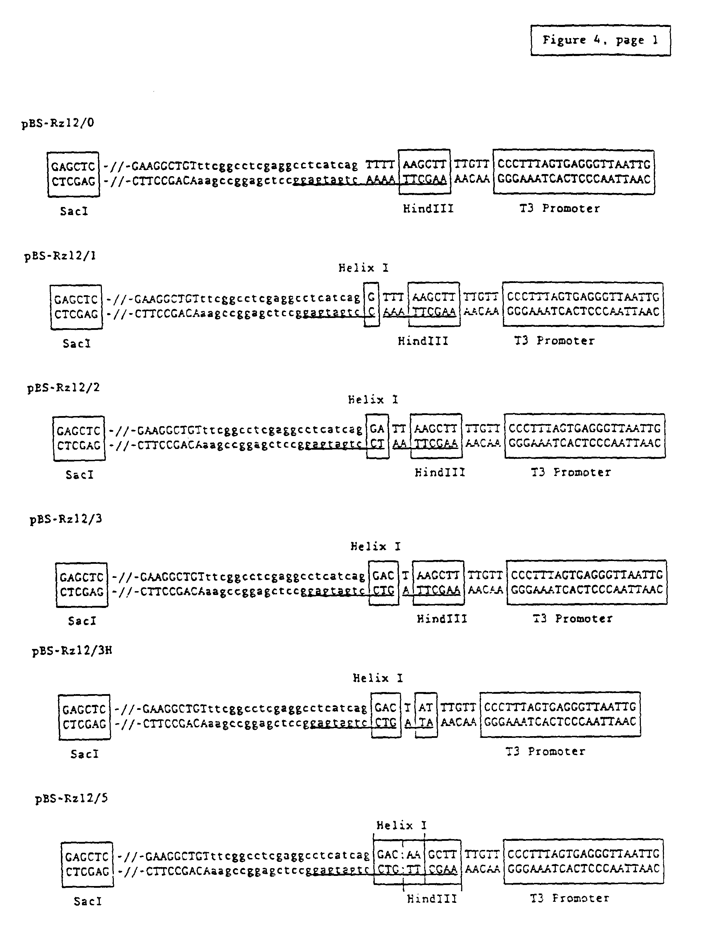

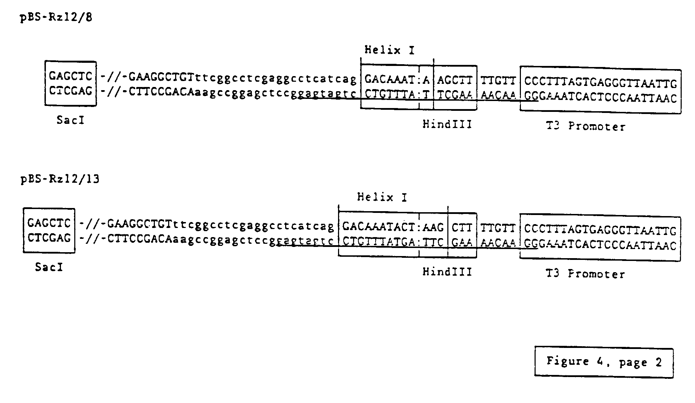

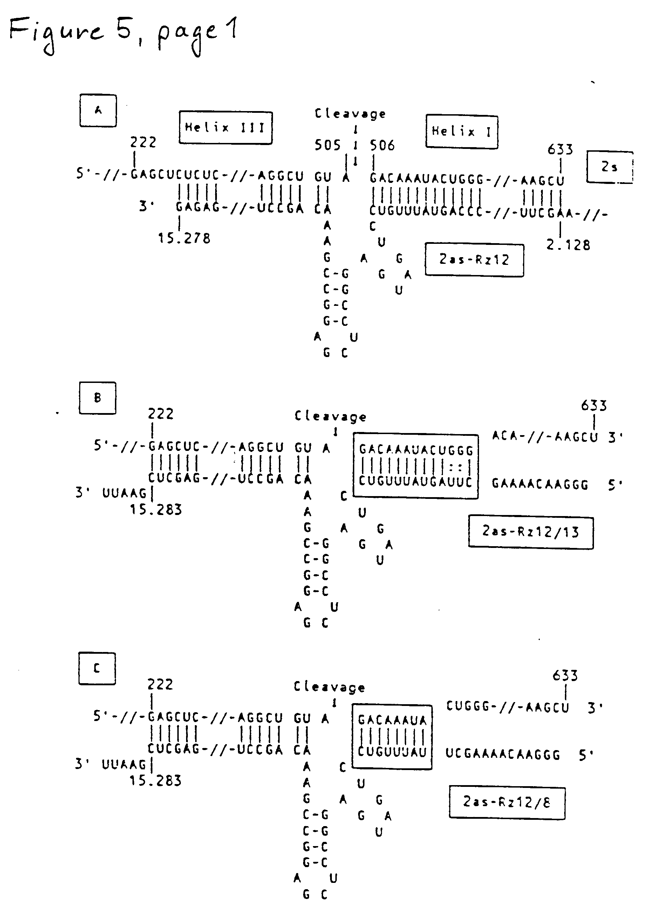

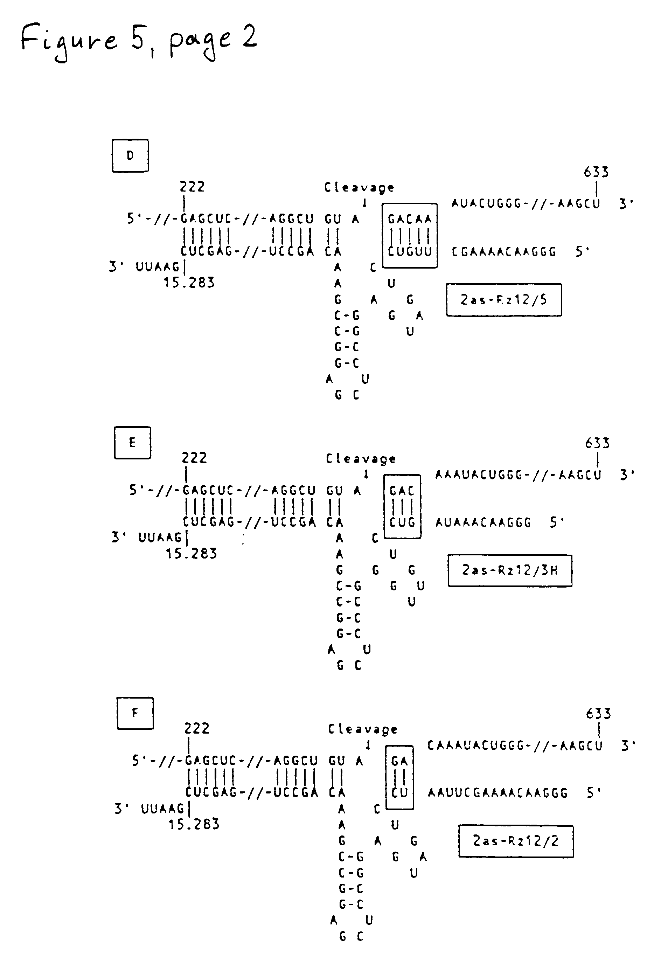

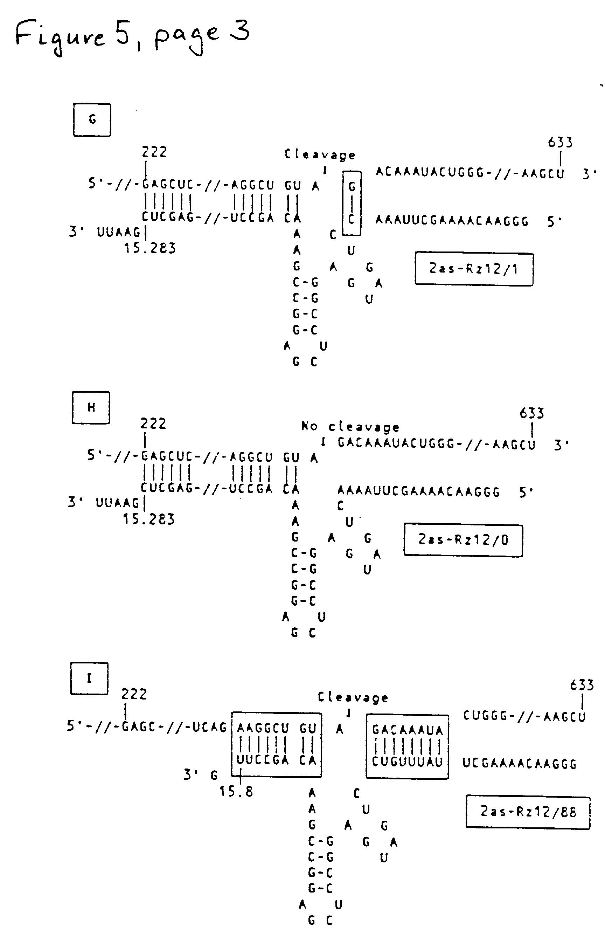

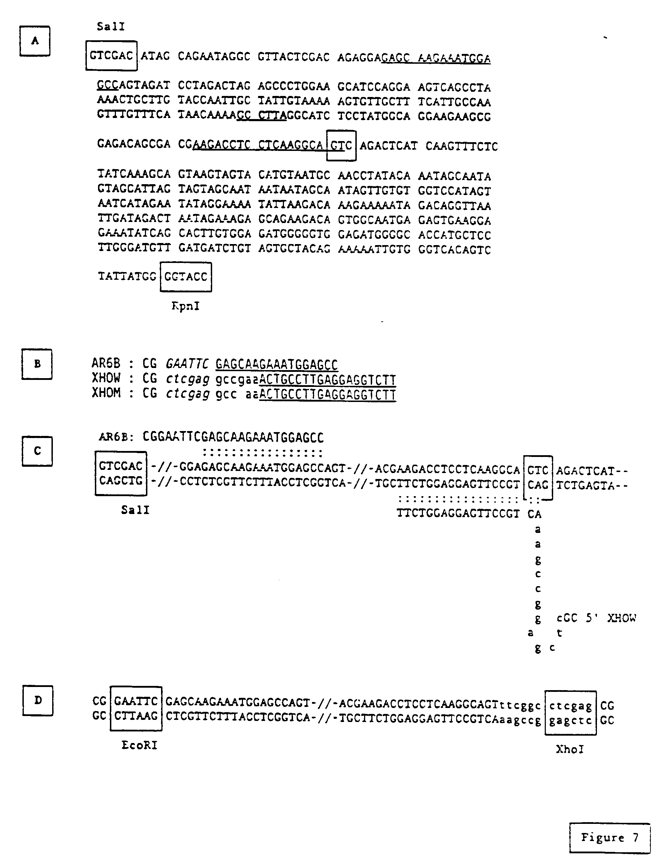

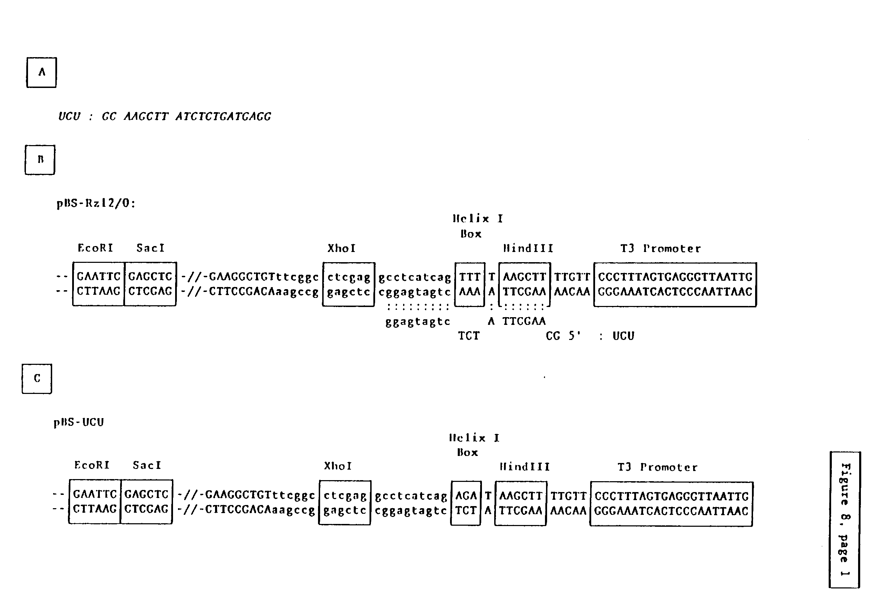

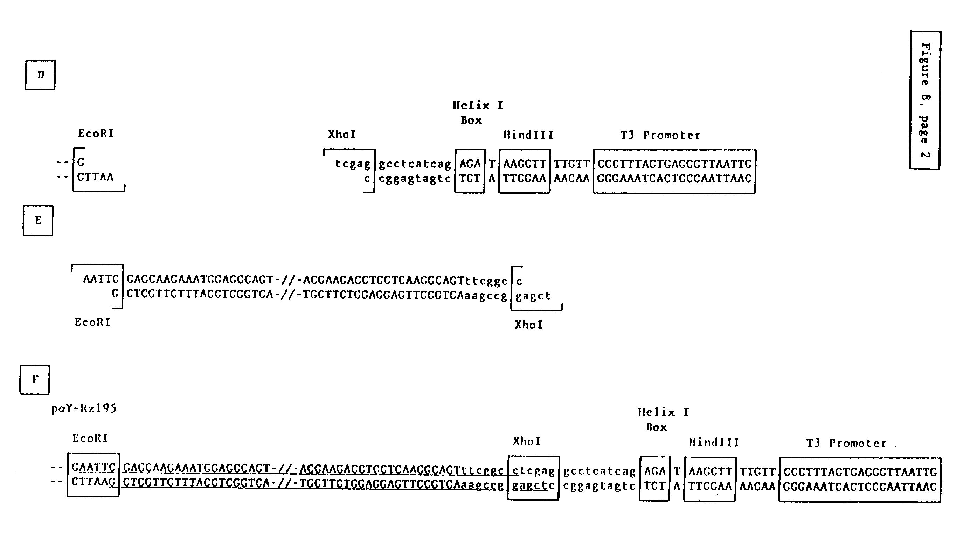

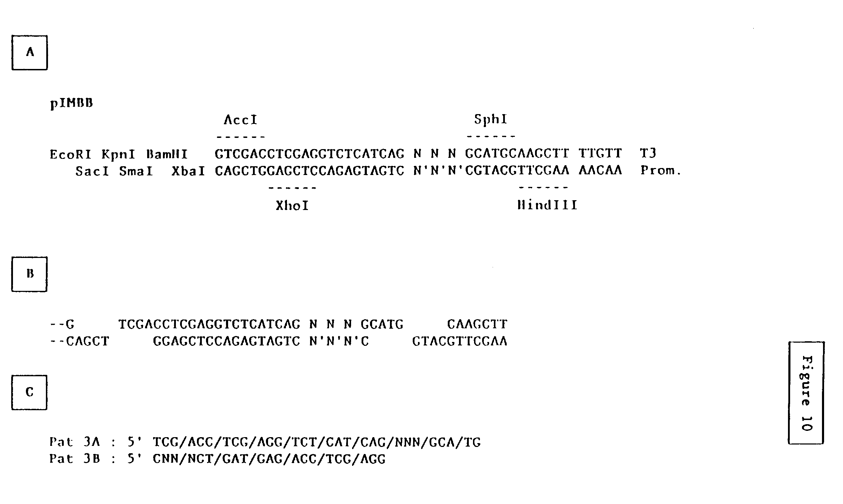

Asymmetric hammerhead ribozymes with a very short Helix I (compare Figures 4 and 5) have at least three major advantages.

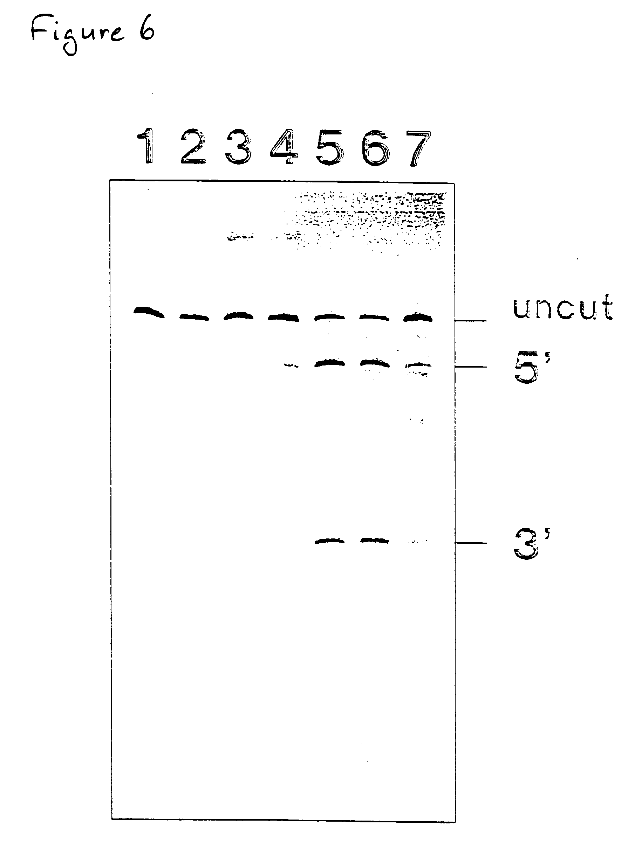

(i) Figure 6 and Example 1 demonstrate that a ribozyme RNA which has a Helix I-forming part of only three or five nucleotides is at least same effective in cleaving the target RNA versus a control that has an extended Helix III of more than 100 nucleotides, which is about as active in cleaving the target RNA as a ribozyme with a two-nucleotide Helix I.
(ii) In such an asymmetric ribozyme with only an extremely short Helix I-forming part of a few nucleotides, the specificity for the target RNA is controlled almost entirely by the Helix III-forming part of the ribozyme. The release of binding energy by the Helix I-formation is extremely low and can be neglected versus the energy release by the Helix III-formation. Such an asymmetric ribozyme can therefore be considered as a modified antisense RNA, corresponding to the Helix III-forming region, which carries at its 5' end a catalytic domain and a very short Helix I-forming region. Such an asymmetric hammerhead ribozyme, resembling a modified antisense RNA, is accessible to techniques developed for determining the 3' terminal extension in antisense RNAs for optimizing binding of antisense RNA to its target (Rittner et al., Nucleic Acids Res. **21** (1993), 1381-1387).
(iii) Since the target-specificity of an asymmetric hammerhead ribozyme arises predominantly from its Helix III-forming region, it allows thirdly different and more simple strategies for generation of target-specific ribozymes. In conventional ribozyme construction two target sequence-specific domains have to be provided: that of the Helix I-and that of the Helix III-forming part. In view of the only 64 conceivable combinations of a three nucleotide motif, it is possible to deliver the Helix I-forming part - later also called "Helix I Box" - and a part or the entire region of the catalytic domain from a set of 64 pre-made DNA molecules which can be contained in a vector. A set of 64 pre-made vectors such as the pUR ("pIMBB") series (Figure 10) or pFORTH series (Figure 17) can be used universally to make all conceivable asymmetric hammerhead ribozvmes without the need to specifically synthesize the sequence of Helix I and a major part of the catalytic domain. Only specific parts corresponding to the Helix III-forming region have to be provided. Once the universal DNAs have been generated the sequences corresponding to the Helix III-forming region are simply attached, generating an asymmetric hammerhead ribozyme. Such universally applicable strategies for generating asymmetric hammerhead ribozymes are outlined in Figures 7-13, 16 and 17.

In a preferred embodiment of the present invention the asymmetric hammerhead ribozyme, the nucleic acid or the nucleotide sequence contains at least one chemically modified nucleotide.
The term "chemically modified nucleotide" refers to nucleotides which differ in their chemical structure from conventional ribonucleotides. The chemical modification can occur in the ribose component, for example 2'-O-methyl, 2'-amino- or 2'-fluororibose or in the internucleotidic phosphate group, which might for example be modified to a phosphorothioate, phosphoramidate, methylphosphonate, phosphotriester, in particular alkylester. Modifications can occur also in any of the four bases or in changes in the stereochemistry (α-nucleotide phophodiester) or by attaching different 5'-terminal groups for example such as psoralen and derivatives, phenandroline and derivatives, ellipicitine and derivatives, EDTA, 5'-p(N-2-chloroethyl-N-methylamino)benzylamide, acridine and derivatives. Also oligonucleotides consisting of mixed ribo- and deoxyribonulceotides are considered as modified.

Another embodiment of the present invention relates to an RNA construct comprising at least two asymmetric hammerhead ribozymes as defined above.
In a preferred embodiment of the present invention the Helix I- and Helix III-forming regions of the asymmetric hammerhead ribozymes of the RNA construct may be same or different.
A specific example of such an RNA construct is shown in Figure 14.

Another embodiment of the present invention relates to a DNA sequence that contains the nucleotide sequence which upon transcription to RNA, corresponds to the asymmetric hammerhead ribozyme or RNA construct, as defined above.
The term "upon transcription" refers to the enzymatic conversion from DNA sequences into RNA sequences by an appropriate RNA polymerase.
Specific examples of such a DNA sequence are shown in Figures 4, 8, 9, 12-14, 16 and 17.

Another embodiment of the present invention relates to a DNA and/or RNA vector comprising the nucleotide sequence, the nucleic acid, the asymmetric hammerhead ribozyme, the RNA construct or the DNA sequence, as defined above.
The term "vector" refers to a DNA and/or RNA replicon that can be used for the amplification of a foreign nucleotide sequence. In particular useful in the context of asymmetric hammerhead ribozymes are DNA or RNA vectors which allow the expression of the hammerhead ribozyme. For example DNA vectors containing the nucleotide sequence encoding for an asymmetric hammerhead ribozyme downstream of an appropriate promoter so that transcription from said promoter generates the functional and catalytically active asymmetric hammerhead ribozyme.

Another embodiment of the present invention relates to a host organism containing at least one of the above defined nucleotide sequences and/or nucleic acids, or at least one of the above defined vector and/or DNA sequence which upon transcription result preferably in a catalytically active asymmetric ribozyme or RNA construct as defined above.

The term "host organism" relates to a virus, bacterium, fungus, plant or mammal.

In a preferred embodiment the present invention relates to a host organism carrying an episomal replication system that is able to amplify the nucleotide sequence encoding an asymmetric hammerhead RNA. The term "episomal replication system" refers to a replication system that is independent of the genome of the host; a plasmid DNA or a virus are episomal replication systems.

In a further preferred embodiment the present invention relates to a genetically engineered host organism containing at least one of the above defined DNA sequence in its genome. The term "genome" refers to the entire genetic material that is inheritable to the next generation. In particular, the present invention relates to organisms which inherit the property to synthesize a functional, catalytically active hammerhead ribozyme.

A further embodiment of the present invention relates to a method for the production of the nucleotide sequences, the nucleic acids, the asymmetric hammerhead ribozymes, the RNA constructs, the DNA sequences or the vectors, as defined above, comprising cultivating the above defined host organism under suitable conditions and isolating the desired products from the culture (cells and/or culture medium) according to procedures known in the art.

A further embodiment of the present invention relates to a process for the preparation of the above defined asymmetric hammerhead ribozymes or RNA constructs.

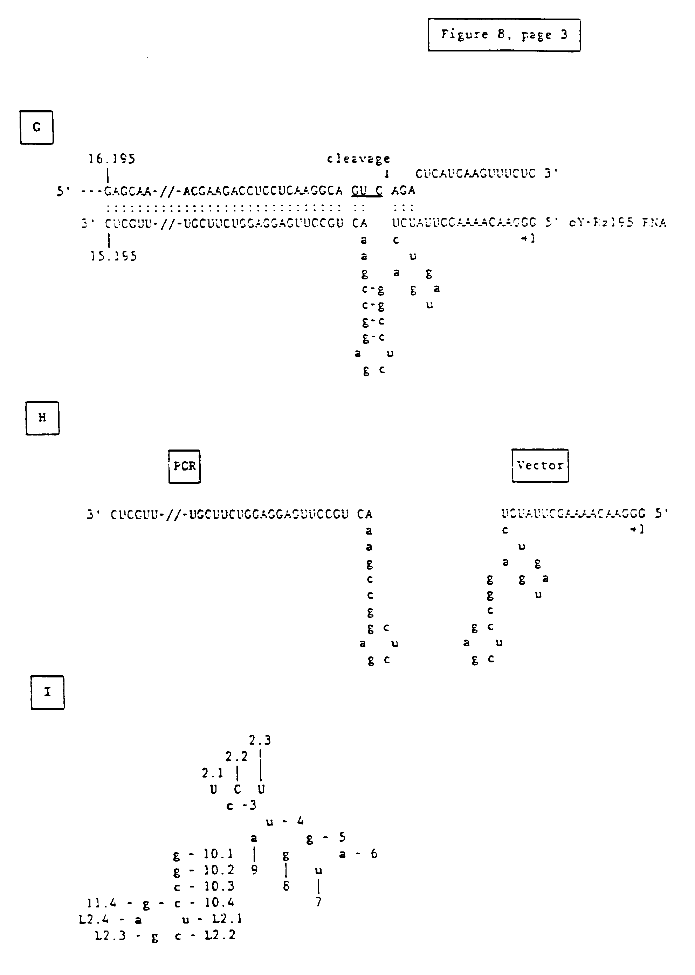

One process of the present invention comprises the steps of generating an asymmetric hammerhead ribozyme or a DNA encoding an asymmetric hammerhead ribozyme by
(i) selecting a cleavable motif in the target RNA,
(ii) selecting the appropriate vector and/or DNA cassette that provides a Helix I Box matching to the selected cleavable motif and a part of the catalytic domain of a hammerhead ribozyme as defined by the general formula 3'-[N]₁₁-[N]_{Lo}-[N]₁₀-[N]₉₋₃- [N] -5' and
(iii) attaching the second sequence that corresponds to the Helix III-forming part and the residual part of the catalytic domain of a hammerhead ribozyme.
The attached second sequence of the present invention that corresponds to the Helix III-forming part and a part of the catalytic domain of a hammerhead ribozyme may be either synthesized chemically or enzymatically or excised by appropriate restriction endonucleases form cDNA that encodes the target RNA.
Specific examples of the method are shown in Figures 8, 9, 12, 13, 16 and 17.

A second process of the present invention comprises the steps of introducing the catalytic domain and the Helix I-forming sequence into DNA by a single PCR step, in which one DNA oligonucleotide provides the sequence of at least part of the catalytic domain and the Helix I-forming region.

A third process of the present invention comprises the step of the chemical synthesis of at least a part of the asymmetric ribozymes.

A fourth process of the present invention relates to the generation of multiple ribozymes by adding a short sense sequence forming an intramolecular Helix III- and Helix I-forming region, including a cleavable motif. Such a process is described in Figure 14.

The above defined nucleotide sequences, nucleic acids and asymmetric hammerhead ribozymes may be either synthesized chemically and/or enzymatically using methods known in the art.

The transcription of the DNA sequences encoding the asymmetric ribozymes of the present invention results in ribozymes capable of inactivating a certain target RNA. Thus, either the DNA sequences encoding the asymmetric ribozymes or RNA constructs of the present invention or the asymmetric ribozymes themselves have extensive therapeutic and biological applications. For example, they can be used for the suppression of any unwanted endogenous genes. They also can be used for the suppression of genes of pathogens or pathogenic RNA not normally present in a cell. Asymmetric hammerhead ribozymes are useful for the treatment of viral infections in man, animals and plant by inactivating a target RNA, produced in the life cycle of the virus. Thus, the asymmetric ribozymes of the present invention can be used as a preventative agent or for the treatment of infections with retroviruses such as the human immunodeficiency virus (HIV) and positive-sense RNA viruses, such as infections with togaviruses, coronaviruses, picornaviruses, caliviviruses and also for infections with negative-sense RNA viruses such as paramyxoviruses (e.g. Sendai virus), rhabdoviruses, the influenca viruses, the bunyaviruses or arenaviruses.

Also the viral mRNAs of DNA viruses can be inactivated by the asymmetric ribozymes of the present invention, so that they can be used for treatment of viral infections with pox-, irido-, herpes- (e.g. herpex simplex virus (HSV)), adeno-, papova- (e.g. hepatitis B virus (HBV)) and/or reoviruses. The asymmetric ribozymes and the corresponding encoding DNA sequences of the present invention can also be used for the inactivation or suppression of target RNAs in procaryotic cells, such as bacteria, or eucaryotic cells such as protozoa and yeast, in plants and animals, such as parasitic animals, e.g. plasmodium fasparum, and mammals, such as humans. In the treatment of humans, the asymmetric ribozymes or RNA constructs of the present invention or the corresponding encoding DNA sequences may be administered to a patient in need thereof.
In plants asymmetric ribozymes may be used for the suppression of any undesired gene for example certain genes that are induced during fruit ripening or during stress caused by heat shock, pathogens, drought, salt or other means. Asymmetric ribozymes will be useful as protective agents against fungal, viral or insect pathogens in transgenic plants or other transformed organisms.

Therefore, the present invention also relates to a composition containing an asymmetric ribozyme, an RNA construct or a corresponding encoding DNA sequence of the present invention or the above defined vector. The present invention furthermore relates to a method of eliminating a target RNA in a plant, an animal or a human patient, which comprises treating said plant, animal or human patient with a DNA sequence encoding the asymmetric ribozyme or the RNA construct of the present invention or with an asymmetric ribozyme of the present invention, optionally in association with a pharmaceutically, veterinarily or agriculturally acceptable carrier and/or excipient. By using such compositions, the asymmetric ribozymes or the corresponding encoding DNA sequences of the present invention may be delivered by parenteral or other means of administration.

In a preferred embodiment of the compositions of the present invention, the DNA sequence encoding the asymmetric ribozyme or the RNA construct of the present invention is contained in a vehicle, such as a carrier virus or a defective interfering particle, by which it is transported to a particular target tissue or cell into the genome of which it can be incorporated or in which it can be transiently expressed. A carrier virus which may be used here for example is a recombinant retrovirus or a recombinant vaccinia virus.

In the case of plants, the compositions of the present invention may contain Ti-plasmid based vectors or vector systems or correspondingly transformed Agrobacteria, which are capable of directing the transfer of the DNA sequences of the present invention into a desired target tissue or target cell of the plant to be treated. Alternatively, the compositions of the present invention may contain expression vectors which are suitable for direct gene transfer techniques into plant cells or tissues, such as electroporation or particle acceleration. Again, the expression of the ribozyme in such a plant target tissue or target cell may be either transient or permanent.

Thus, the present invention also relates to compositions for eliminating the disease-causing capability of an infectious agent such as viroids, virus, bacteria, protozoa or fungus.

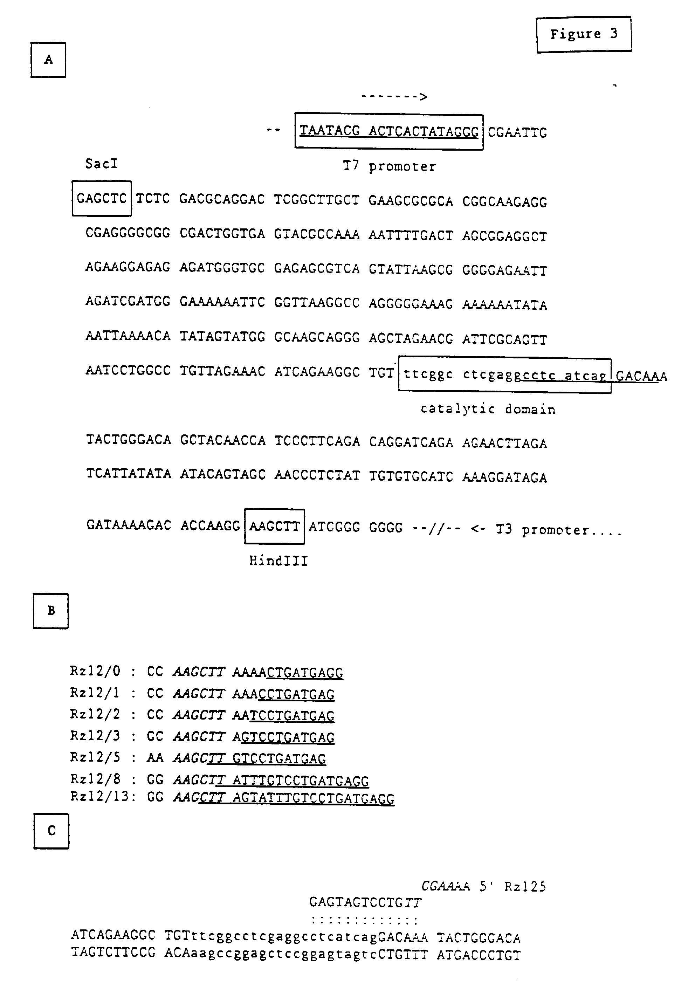

The Figures show:
Figure 1
The hammerhead complex formed between the target RNA, which is HIV-1 RNA and the ribozyme RNA 2as-Rz12 which has been described (Homann et al., Nucleic Acids Res. 21 (1993), 2809-2814). The cleavable motif GUA within the HIV-1 RNA is underlined and the catalytic domain is given in lower case letters. The numbers refer to nucleotide numbers of the RNA 2s also described in Hertel et al., supra. The A nucleotide of the cleavable motif corresponds to position 506 and the complementary regions between substrate and ribozyme RNA range from 227 till 633, resulting in a double-stranded region of 278 nucleotides 5' of the cleavable motif and 128 nts 3' (downstream) of it.
For details how this example of a ribozyme substrate complex in the hammerhead conformation matches the numbering system of hammerhead RNAs according to the convention (Hertel et al., supra), see Figure 2.
Figure 2
The same hammerhead ribozyme-substrate complex as in Figure 1 is outlined. The cleavable motif GUA of the target RNA (nucleotides 16.2, 16.1, 17) is underlined and the catalytic domain (ranging from nucleotide 3 till 14) is given in lower case letters; these nucleotides do not interact by base-pairing with the target RNA. It should be stressed, however, that the term "catalytic domain" is not completely precise, since also nucleotides 15.1 and 15.2 are essential for catalytic activity; in this application, nucleotides 15.1 and 15.2 are not included in what is expressed by "catalytic domain".
Helix I is formed by the two complementary sequences of target RNA and ribozyme RNA, respectively, ranging from nucleotides 1.1 till 1.x (5' to 3') and 2.1 till 2.x (3' to 5'), wherein in this example x is 128.
Helix III is formed by the two complementary sequences of target RNA and ribozyme RNA, respectively, ranging from nucleotides 16.1 till 16.y (3' to 5') and 15.1 till 15.y (5' to 3'), wherein in this example y is 278.
Helix II is intramolecularly formed within the catalytic domain by the nucleotides 10.1 till 10.4 (5' to 3') and the complementary nucleotides 11.4 till 11.1 (3' to 5').
The Helix II-forming nucleotides are connected by the four nucleotides L2.1 till L2.4 which form a hairpin loop.
In this particular example two recognition sequences for restriction enzymes are contained within the catalytic domain: one for StuI AGG/CCT (nucleotides 9 till L2.1) and XhoI C/TCGAG nucleotides 10.4 till 11.4.
Figure 3
DNA sequence of pBS29-Rz12 and the DNA oligodeoxynucleotides used for generating a DNA construct encoding for an asymmetric hammerhead ribozyme. In panel (A) the relevant sequence of plasmid pBS29-Rz12 is detailed. Only one DNA strand is given. It is the strand that is of complementary polarity versus the ribozyme sequence 2as-Rz12 and of the same polarity as the HIV-1 target RNA. The HIV-1-specific sequence is contained between the SacI and HindIII sites which are boxed. The first G nucleotide of the SacI recognition sequence GAGCT/C corresponds to nucleotide complementary to nucleotide 15.y or 15.278 in Figure 2 and Figure 5. The last T nucleotide of the HindIII recognition sequence A/AGCTT corresponds to nucleotide 2.x or 2.128. The sequence that is complementary to the catalytic domain is given in lower case letters and is boxed, wherein the first T nucleotide corresponds to the nucleotide that is complementary to nucleotide 14 of Figure 2 and the last G nucleotide is complementary to nucleotide 3 of Figure 2. The sequence of the T7 promoter is detailed (see box) and the direction of transcription is indicated, whereas the position and the direction of the T3 promoter is schematically indicated.
The underlined sequences represent regions where the DNA oligonucleotides could bind to the pBS29-Rz12 DNA (compare description of Example 1). The "T7 primer" matches exactly to the T7 promoter region. The region that is covered by at least one of the DNA oligos Rz12/0 - Rz12/13 is also underlined.
The plasmid pBS29-CX, which was used to generate labeled HIV-1 target RNA contains instead of the catalytic domain (boxed sequence) an additional A residue.
(B) shows the sequences of the DNA oligonucleotides that have been used for the PCR experiments described under Example 1. The region within the DNA oligonucleotides that is able to bind to pBS29-Rz12 is underlined and the HindIII recognition sequence that is contained in the 5' region of each of the DNA oligonucleotides is indicated and given in italics.
(C) gives a more detailed example how the oligonucleotide Rz12/5 binds to the pBS29-Rz12 DNA, which is here given as a double-strand.
Figure 4
DNA sequence of the plasmids pBS-Rz12/0, pBS-Rz12/1, pBS-Rz12/2, pBS-Rz12/3, pBS-Rz12/3H, pBS-Rz12/5, pBS-Rz12/8, pBS-Rz12/13, which encode asymmetric hammerhead ribozymes. The relevant sequence of the plasmids obtained by PCR as described in Example 1 are given. The DNA sequence corresponding to the catalytic domain is given in lower case letters. The HindIII and SacI sites used for cloning into pT3T71ac (Boehringer Mannheim) are boxed, as well as the T3 promoter from which the corresponding asymmetric hammerhead ribozymes are transcribed. The sequences originating from the DNA oligonucleotides used in the PCR reactions are underlined (compare also Figure 3A, B and C). The sequence that is not detailed but given as -//- can be deduced from Figure 3A.
Figure 5
Ribozyme-substrate complex formed between HIV-1 target RNA and the asymmetric hammerhead ribozymes 2as-Rz12/0, 2as-Rz12/1, 2as-Rz12/2, 2as-Rz12/3H, 2as-Rz12/5, 2as-Rz12/8, 2as-Rz12/13, 2as-Rz12/88 .
After transcription of EcoRI-linearized plasmids pBS-Rz12/0, pBS-Rz12/1, pBS-Rz12/2, pBS-Rz12/3H, pBS-Rz12/5, pBS-Rz12/8, pBS-Rz12/13, pBS-Rz12/88 with T3 RNA polymerase, the following asymmetric hammerhead ribozymes 2as-Rz12/0, 2as-Rz12/1, 2as-Rz12/2, 2as-Rz12/3H, 2as-Rz12/5, 2as-Rz12/8, 2as-Rz12/13, 2as-Rz12/88, respectively are synthesized. When forming a hammerhead complex with the HIV-1 target RNA the structures given are obtained. The sequence corresponding to the catalytic domain is given in lower case letters. Helix III remains the same as in Figures 1 and 2 and consists of 283 nucleotides. Helix I is boxed. Helix I corresponds to [N] of the general formula 3'-[N]₁₁-[N]_{Lo}-[N]₁₀-[N]₉₋₃- [N] -5', wherein x corresponds to the length of Helix I. In these examples x is 0, 1, 2, 3, 5, 8, 13 for 2as-Rz12/0, 2as-Rz12/1, 2as-Rz12/2, 2as-Rz12/3, 2as-Rz12/5, 2as-Rz12/8, 2as-Rz12/13, respectively. The difference in size between Helix I and Helix III is reflected by the term "asymmetric hammerhead ribozyme".
Figure 6
Ribozyme assay of the asymmetric hammerhead ribozymes 2as-Rz12/0, 2as-Rz12/1, 2as-Rz12/2, 2as-Rz12/3, 2as-Rz12/5.
HindIII-linearized plasmid pBS29-CX was used for the synthesis of radioactively labeled HIV-1 target RNA. About 30 fmole of radioactively labeled target RNA was incubated in 20 mM MgCl₂, 50 mM Tris/HCl, pH 8.0 for 60 minutes at 37°C, either alone (lane 1) or in combination with a 10 fold molar excess of the asymmetric hammerhead ribozymes 2as-Rz12/0 (lane 2), 2as-Rz12/1 (lane 3), 2as-Rz12/2 (lane 4), 2as-Rz12/3 (lane 5), 2as-Rz12/5 (lane 6) or with the original ribozyme RNA 2as-Rz12 (lane 7). The reaction products were separated on a 5% polyacrylamide gel containing 8 M urea and visualized by autoradiography. The position of the 5' and 3' cleavage products is indicated. It can be seen that asymmetric hammerhead ribozymes having a Helix I of 3 or 5 bases (2as-Rz12/3 or 2as-Rz12/5 are much more effective in cleaving the target RNA than the conventional hammerhead ribozyme 2as-Rz12. Even the asymmetric hammerhead ribozyme 2as-Rz12/2 shows a considerable cleavage activity comparable to that of 2as-Rz12/5. Asymmetric hammerhead ribozyme in which Helix I is missing (2as-Rz12/0) or consists of only one nucleotide (2as-Rz12/1) have either no or only very limited cleavage activity.
The positions corresponding to the cleaved (5' and 3' cleavage product) and uncut materials in lanes 5-7 were excised from the gel and the radioactivity contained, therein was determined in counts per minute as follows:

| [cpm] | | | | | |
|---|---|---|---|---|---|
| | uncut | 5' | 3' | total | cleaved RNA/total RNA in % |
| 2as-Rz12/3 | 940 | 1183 | 799 | 2922 | 67.8 |
| 2as-Rz12/5 | 837 | 1022 | 693 | 2552 | 67.2 |
| 2as-Rz12 | 2204 | 382 | 229 | 2815 | 21.7 |

A turnover of 67% after one hour corresponds to a half-life of the target RNA of about 35 minutes when incubated with either of the asymmetric hammerhead ribozymes 2as-Rz12/3 or 2as-Rz12/5, whereas a turnover of about 22% obtained with the conventional hammerhead ribozyme 2as-Rz12 corresponds to a half-life of about 160 minutes. From these data it can be concluded that asymmetric hammerhead ribozymes are about 5 times more effective in catalytic activity compared to conventional hammerhead ribozymes with long Helix I. Given the fact that the lag time necessary for forming the double-stranded complex, which is essential for ribozyme cleavage was not considered in this experiment the actual difference in the cleavage rates is expected to be much higher.
Figure 7
Sequence of the HIV-1 cDNA insert of pAR6 and DNA oligonucleotides used for PCR amplification in order to generate an asymmetric hammerhead ribozyme. Part A shows the cDNA 564 base-pair cDNA insert between nucleotides 5819-6382 of the Human T-cell lymphotropic virus type III. The strand given corresponds to the sense strand, i.e. the RNA which can be targeted by an asymmetric hammerhead ribozyme is of the same polarity. The SalI and KpnI sequences are boxed, as well as the GUC sequence that was selected as cleavable motif. The underlined sequences correspond to the binding sites for DNA oligonucleotides pAR6 and XHOW used in the PCR reaction described under Example 2. The underlined sequence GCCTTA, which is given in bold, is mentioned in Figure 14. Part B shows the sequence of these two DNA oligonucleotides and a third DNA oligonucleotide XHOM which is missing a G residue compared to XHOW and which was used in a further PCR experiment to generate a catalytically inactive asymmetric hammerhead ribozyme αY195inac. The underlined parts are able to bind to the pAR6 DNA, the two restriction recognition sequences for EcoRI G/AATTC and XhoI C/TCGAG are indicated and given in italics. Sequences corresponding to the catalytic domain of asymmetric hammerhead ribozymes are given in lower case letters. Part C show the two DNA oligonucleotides bound to the pAR6 DNA, wherein the XHOW DNA oligonucleotide is given in the conformation of the RNA of a hammerhead ribozyme for reasons of clarity only.
The sequence of the amplified PCR product is given in part D.
Figure 8
Generation of the asymmetric hammerhead ribozyme αY-Rz195 by recombining a PCR-produced DNA with a plasmid DNA having the appropriate Helix I Box.
Part A gives the sequence of the DNA oligonucleotide UCU, wherein the HindIII sequence A/AGCTT is given in italics.
Part B shows the binding site to plasmid pBS-Rz12/0, which has an AAA Helix I Box. The UCU DNA oligonucleotide is not complementary in the region of the Helix I Box.
Part C shows the plasmid obtained after re-cloning the PCR product obtained using the UCU DNA oligonucleotide and the T7 "primer" DNA oligonucleotide into pT3T71ac. The resulting plasmid pBS-UCU has a TCT Helix I Box.
Part D shows the plasmid pBS-UCU after cleavage with EcoRI and XhoI and part E illustrates the PCR product given in Figure 7D cleaved with EcoRI and XhoI. Ligation of the two DNAs resulted in pαY-Rz195 given in part F, wherein the region derived from the PCR product is underlined.
Part G shows the asymmetric hammerhead complex formed between the αY-Rz195 RNA and the HIV-1 target RNA, having a Helix I of only three nucleotides.
Part H shows schematically which regions of the asymmetric hammerhead ribozyme are derived from the PCR- produced DNA and which had been delivered by the pBS-UCU vector.
The regions contained in the pBS-UCU vector is also given in part I which gives also the nucleotide numbers according to the convention (Hertel et al., supra). This sequence, which itself is not a functional hammerhead ribozyme, is a specific example of the general formula 3'-[N]₁₁-[N]_{Lo}-[N]₁₀-[N]₉₋₃-[N]-5' given in the text and in claim 1.
In this specific example, [N] corresponds to 5' UCU (TCT) representing Helix I in a hammerhead ribozyme/target-complex (or the Helix I Box in case of DNA, compare Figure 8C), [N]₉₋₃ corresponds to 5' CUGAUGA, representing nucleotides 3 to 9 of a hammerhead ribozyme, [N]₁₀ corresponds to 5' GGCC representing nucleotides 10.1 till 10.4 (corresponding to 10.n, n being 4) and [N]₁₁ represents G or nucleotide 11.4 (corresponding to 11.m, m being 4) and [N]_{Lo} corresponds to 5' UCGA representing the nucleotides L2.1, L2.2, L2.3, L2.4 capable of forming Loop 2 of a hammerhead ribozyme.
The restriction recognition sequence used for recombination with the PCR product is XhoI (C/TCGAG) (nucleotides 10.4 till 11.4). Also a second restriction recognition sequence is present: StuI (AGG/CCT) (nucleotides 9 till L2.1). In principle it is also conceivable that recombination proceeds via the latter restriction site so that it is possible that [N]₁₁ or even [N]_{Lo} are missing in a vector having a Helix I Box.
Figure 9
A second strategy for generation of asymmetric hammerhead ribozymes by recombining a PCR-produced DNA with a plasmid DNA having the appropriate Helix I Box.
Part A shows the parts of the asymmetric hammerhead ribozyme that have to be delivered by PCR and the parts that originate from a particular vector DNA chosen of pre-made set of 64 vectors containing each a different three-nucleotide Helix I Box. Compared to the first strategy outlined in Figure 8, the sequence of the catalytic domain has been changed in the position of nucleotides 10.2 and 11.2 (given in upper case letters). Further the part of the sequence corresponding to the catalytic domain that is contained in the vector has been extended so that recombination proceeds via nucleotides 11.1 and 12.
Part B shows the sequence of such a vector. It contains two restriction sites A and B, wherein each A and B can be also a set of sites and B is also dispensable. The Helix I Box consists of three nucleotides, which can be any of the 64 possible combinations and it contains an AccI recognition site GT/CGAC wherein the CGAC part corresponds to nucleotides 12, 11.1, 11.2, 11.3.
The PCR product necessary for recombination into an DNA encoding an asymmetric hammerhead ribozyme (part C) contains a terminal SfuI site (TT/GCAA) wherein the TT/GA part corresponds to nucleotides 14, 13, 12, 11.1. The SfuI site is followed by two or more unrelated nucleotides N and N' which facilitate or support cleavability by SfuI. After cleavage of the DNAs with a restriction endonuclease that cleaves in each region A and with SfuI or AccI, respectively, (part D), the two DNA become recombined (part E), resulting is a destroyed AccI and SfuI site. The RNA transcribed form the upstream promoter delivers an asymmetric hammerhead ribozyme that is given in part F.
Figure 10
The pUR series of vectors, usable for the strategy for generation of asymmetric hammerhead ribozymes described in Figure 9.
Part A shows the universal vector pUR for generating ribozymes. It contains the sequence of the catalytic domain of hammerhead ribozymes as outlined in Figure 9, inserted in the SalI (AccI) and SphI sites of the plasmid pT3T71ac (Boehringer Mannheim, Germany). The Helix I Box is given by NNN and N'N'N', which can be any of the 64 possible combinations. For example in pUR-AAA the sequence N'N'N' would be AAA, which would be appropriate for construction of an asymmetric hammerhead ribozyme requiring a Helix I consisting of the sequence AAA. The set of plasmids was obtained by inserting a synthetic DNA cassette (part B) into the pT3T71ac cleaved with SalI and SphI. The cassette consisted of the two DNA oligonucleotides Pat3A and Pat3B which are shown in part C.
Figure 11
Generation of a vector with a 4 nucleotide Helix I Box by insertion of a synthetic DNA cassette.
Part A shows schematically the polylinker region of the plasmid pBluescript II KS (Stratagene) with the XhoI and PstI sites detailed. The two DNA oligonucleotides HB1 and HB2 were phosphorylated, annealed and inserted into the plasmid cut with XhoI and PstI (part B). Part C shows the final vector pAGUG with the AGTG Helix I Box. Also vectors with longer Helix I Boxes could be constructed in such a way.
Figure 12
Generation of an asymmetric hammerhead ribozyme by insertion of a cDNA cassette into an EcoRI site.
Part A shows the hammerhead complex of an asymmetric hammerhead ribozyme with a target sequence containing the sequence GAAUUC which corresponds at the cDNA level to an EcoRI restriction recognition sequence.
Part B shows the cDNA that encodes the target RNA and part C demonstrates how a synthetic DNA cassette can be inserted into the EcoRI-cleaved cDNA, thereby generating a DNA that encodes for an asymmetric hammerhead ribozyme. The nucleotide sequences illustrated with N and N' can contain any other useful sequence such as additional restriction recognition sequences and/or a promoter region.

Figure 13
Generation of an asymmetric hammerhead ribozyme by insertion a cDNA into a pre-made vector containing a Helix I Box.
Part A shows a vector DNA consisting of one or several restriction recognition sequences A, and an ApoI restriction recognition sequence and the catalytic domain of a hammerhead RNA (in lower case letters), followed by a Helix I Box and optional restriction sites B and an optional promoter.
Part B symbolized a cDNA having restriction recognition sequences for any restrictions site A, that is present also in the vector in part A and EcoRI. After digestion of the cDNA and the vector, having the matching Helix I Box, with the restriction enzyme A and EcoRI or ApoI, respectively, the cDNA fragment is ligated into the vector (part C) generating a DNA that encodes an asymmetric hammerhead ribozyme (part D). The asymmetric hammerhead ribozyme is directed against the UUC motif that is part of the EcoRI restriction recognition sequence (part E).

Figure 14
Generation of a double ribozyme containing an intramolecular self-cleavage site.
Part A shows the asymmetric hammerhead complex formed between the RNA αY-Rz195 (compare Figures 7 and 8) with its target RNA. Nucleotides 15.4 till 15.9 and nucleotides 15.58 till 15.63 are underlined. These sequences are complementary.
Part B shows the intramolecular interaction between these two hexanucleotide sequences.
Part C shows the position of the nucleotides 15.58 till 15.63 within the plasmid pαY-Rz195. The sequence corresponding to the catalytic domain is given in lower case letters. The binding sites for the two DNA oligonucleotides DRZ and XHOW (see D) are underlined. Plasmid pαY-Rz195 contains a HindIII restriction recognition sequence (boxed) located between the T3 Promoter and the Helix I Box. This HindIII site was destroyed by digestion with HindIII, followed by treatment with nuclease S1 and re-ligation which delivered plasmid pαY-Rz195-H (part D). This plasmid was used as template for a PCR reaction carried out with the two DNA oligonucleotides DRZ and XHOW which are given. DRZ contains a restriction recognition sequence for EcoRI and HindIII. The resulting PCR product was cleaved with EcoRI and XhoI and re-inserted into pαY-Rz195-H yielding plasmid pαY-Rz63 (part E). This plasmid encodes the RNA αY-Rz63 which is able to form the intramolecular asymmetric hammerhead complex given in part F. The nucleotides 15.58 till 15.63 can now be considered as nucleotides 16.9 to 16.4 of an intramolecular hammerhead RNA.
After insertion of the EcoRI-HindIII fragment of plasmid pBS-Rz12/3 (Figure 4) into the EcoRI and HindIII site of paY-Rz63 a DNA is generated that encodes a double ribozyme. The RNA transcript of this DNA is shown in part G. The RNA consists of two domains, the aY-Rz63 domain (bottom) which is located at the 5' terminus and connected by the sequence AAGCUU (boxed), which represents the restriction recognition sequence for HindIII, to the 3'-terminal domain resembling the 2as-Rz123 RNA. Since the 5'-terminal domain can assume the intramolecular conformation of an asymmetric hammerhead ribozyme, cleavage occurs at the site indicated, releasing the two RNA shown in part H. The 3'-terminal C nucleotide of the αY-Rz63 domain carries a 2',3'-cyclophosphate. Both RNAs will independently cleave their HIV-1 target RNA at different sites.
Figure 15
Schematic maps of the HIV-1 target sequence and the catalytic antisense RNAs.
The top shows functional elements of the HIV-1 genome and the solid bar below details the region between nts. 222-633 which correspond to the sense RNA 2s that was used as target RNA. Functional elements within this region are also indicated. The antisense RNA 2as is complementary to RNA 2s. The catalytic antisense RNA 2as-Rz12 contains an incorporated catalytic domain of 22 nts, which causes cleavage of the target RNA between nts. 505 and 506. The bar at the bottom shows the catalytic antisense RNAs with truncated helices I.
Figure 16
Strategy for simplified generation of asymmetric hammerhead ribozymes.
The left panel shows the conversion of the plasmid pBS-Rz12/0 that encodes - if transcribed from the T3 promoter - the catalytic domain of a hammerhead ribozyme preceded by an AAA sequence that could form a Helix I in an asymmetric hammerhead ribozyme. By using the DNA oligonucleotide UCU and the T7 sequencing primer d(TAATACGACTCACTATAGGG), a DNA fragment was PCR-amplified and re-cloned, resulting in plasmid pBS-UCU with a modified Helix I box. The resulting T3 RNA transcript could pair to a target RNA having a cleavable motif that is followed by an AGA sequence. Similarly, all conceivable three-nucleotide Helix I boxes can be introduced. The right panel shows the conversion of the plasmid pRC-SR6, that contains HIV-1 cDNA sequences [nucleotide numbers refer to sequence of Ratner et al., Nature, **313** (1985), 277-284; those in parenthesis to the SR6 RNA transcript], into a ribozyme construct. The GTC sequence corresponding to the selected target site is boxed. Two nested DNA oligonucleotides AR6B and XHOW were used for PCR amplification. The latter one is given in the conformation of a hammerhead structure for clarity only. The amplified DNA was cloned via the newly introduced EcoRI and XhoI sites into the plasmid pBS-UCU that provides the matching Helix I box, yielding plasmid pαY195. The related control plasmid pαY195inac was generated in a similar way, by using instead of XHOW a DNA oligo without the G residue that proceeds the AAA sequence at the 3' terminus of the catalytic domain. After transcription with T3 RNA polymerase, the asymmetric ribozyme RNA αY195 (or αY195inac) is generated which is shown below in its hammerhead conformation together with the target RNA SR6. This hammerhead complex forms a three-nucleotide Helix I and a Helix III consisting of 195 nucleotides. Thus construction of asymmetric hammerhead ribozymes just requires PCR amplification, followed by subcloning into the matching vector that delivers the catalytic domain plus the target-specific Helix I-forming region.
Figure 17
A third strategy for generation of asymmetric hammerhead ribozymes by recombining a PCR-produced DNA with a plasmid DNA with the appropriate Helix I Box.
Part A shows the parts of the asymmetric hammerhead ribozyme that have to be delivered by PCR and by a set of pre-made vectors containing a Helix I Bcx. Compared to the first strategy outlined in Figure 8, the sequence of the catalytic domain has been changed in the position of nucleotides L2.2 and L2.3 (these changes are not of particular importance) and at positions L2.4 (C instead of G). As a result, a new EagI site (C/GGCCG) is created which is given in capital letters comprising nucleotides L2.4, 11.4-11.1 and 12.
Part B shows the sequence of such a vector (pFORTH series). It contains two restrictions sites A and B, wherein each A and B can be also a set of sites and B is also dispensable. The Helix I Box consists of three nucleotides, which can be any of the 64 possible combinations.
The PCR product necessary for recombination into an DNA encoding an asymmetric hammerhead ribozyme (part C) contains a terminal EagI site (C/GGCCG) corresponding also to nucleotides L2.4, 11.4-11.1 and 12.
After cleavage of the DNAs with EagI and a restriction endonuclease that cleaves in each region A (part D) the two DNA become recombined (part E), resulting is a regenerated EagI site. The RNA transcribed form the upstream promoter delivers an asymmetric hammerhead ribozyme that is given in part F.
Figure 18.
Cleavage analysis of a set of 2as-derived catalytic antisense RNAs.
**(A)** Radiolabelled target RNA 2s was incubated for 5 minutes at 37°C alone (lane 1) or with the ribozyme RNAs indicated on top of each lane and separated on a denaturing polyacrylamide gel. Lane M contains radiolabelled HinfI fragments of plasmid pBR322. The position of the substrate RNA (S) and the position of the 5' and 3' cleavage products are indicated.
**(B)** The same reaction as in (A), except, incubated for 60 minutes. The RNAs analyzed in lane 11 were pre-heated for 5 minutes at 60°C followed by chilling in ice and normal incubation at 37°C for 60 minutes.

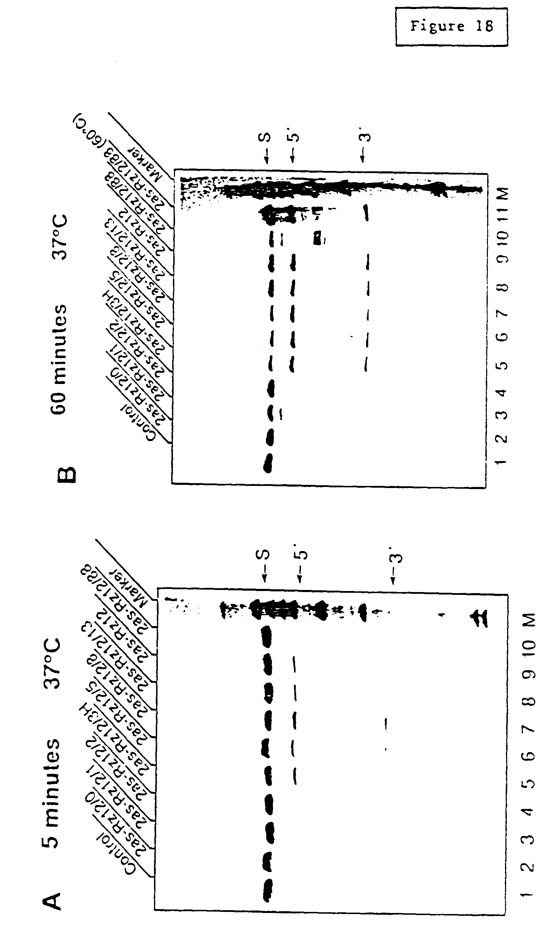

Figure 19.
Analysis of the ribozyme reactions on a native polyacrylamide gel.
The ribozyme reaction was carried out under condition as described in the legend to Figure 18B and the reaction products were separated on a native polyacrylamide gel. The position of the substrate RNA is indicated (S). Substrate RNA that binds ribozyme RNA forms a complex that does not migrate into the gel (C). Ribozyme RNAs analyzed in lanes 4-8 release the 3' cleavage product (3'). Lane M contains radiolabelled HinfI fragments of plasmid pBR322.
Figure 20
Replication of HIV-1 in the presence of asymmetric hammerhead ribozymes and control RNAs.
All RNAs (160 ng) were tested by co-transfection in human SW480 cells with infectious proviral HIV-1 DNA (pNL4-3, 40 ng). CAT RNA served as control 100% replication. The bars represent averages of 24 measurements (16 in case of αY-Rz195 and αY-Rz195inac); the average values in % replication are given in numbers next to the bars and the standard deviations of the mean values are indicated in the diagram. Black bars represent catalytically inactive RNAs and dotted bars represent catalytically active RNAs.
Figure 21
Cleavage of RNA SR6 by the asymmetric hammerhead ribozyme αY-Rz195.
Radioactively labelled substrate RNA SR6 was incubated for different reaction times as indicated on top of each lane (in hours) with the asymmetric hammerhead ribozyme αY-Rz195 and analyzed on a polyacrylamide/urea gel and visualized by autoradiography. The position of the substrate (S) and of the 5' and 3' cleavage products are indicated.

The following examples illustrate the invention:

More information on the methods applied herein can be found in Sambrook et al., "Molecular Cloning", Cold Spring Harbor, second edition, 1989.

### Example 1: Generation of asymmetric hammerhead ribozymes

The plasmid pBS29-Rz12 encodes for the HIV-1-specific ribozyme 2as-Rz12 that is given in Figures 1, 2 and 15. The relevant sequence of plasmid pBS29-Rz12 is shown in Figure 3 and also the DNA sequence encoding the catalytic domain is indicated as well as the position of T3 and T7 promoters and the position of the SacI and HindIII restriction sites.
In order to generate asymmetric ribozymes which are able to form only a short Helix I, the DNA of plasmid pBS29-Rz12 was subjected to several polymerase chain reactions (PCR).
Initially two reactions were done each in a reaction volume of 100 µl containing:
20 mM Tris pH 8.3, 1.5 mM MgCl₂, 25 mM KCl, 0.05 % Tween 20, bovine serum albumin (molecular biology grade) 0.1 mg/ml, a mixture of dATP, dCTP, dGTP, dTTP, each 0.1 mM, 100 pg of plasmid DNA of pBS29-Rz12, 20 pmole of the oligodeoxynucleotide ("T7 Primer") d(TAATACGACTCACTATAGGG) and 20 pmole of either the oligonucleotide Rz12/5 d (AAAAGCTTGTCCTGATGAG) or Rz12/3 d (GCAAGCTTAGTCCTGATGAG), respectively, and 5 units of Taq DNA polymerase (Minotech, Heraklion/ Crete, Greece.
The reaction was performed in a DNA Thermal Cycler (Perkin Elmer Cetus) according to the reaction scheme:
7 minutes at 94°C, four cycles consisting of 30 seconds at 94°C, 30 seconds at 15°C, 2 minutes at 72°C. 30 cycles followed consisting of 30 seconds at 94°C, 30 seconds at 45°C, 2 minutes at 72°C, before the reaction mixtures were incubated for a final 7 minutes at 72°C.
The PCR products were cleaved with SacI and HindIII and ligated into the vector pT3T71ac (Boehringer Mannheim) at the same sites.
The resulting plasmids were called pBS-Rz12/3 and pBS-Rz12/5, respectively. Their relevant sequence was confirmed by dideoxysequencing. The sequences of the DNA is shown in
Figure 4 and the RNA transcripts obtained by T3 RNA polymerase from pBS-Rz12/3 and pBS-Rz12/5 are outlined in 5 where the conformation of the resulting asymmetric hammerhead ribozyme RNAs is indicated. The plasmid pBS-Rz12/3 was digested with HindIII and treated with nuclease S1 to trim the protruding ends. Re-ligation generated plasmid pBS-Rz12/3H.

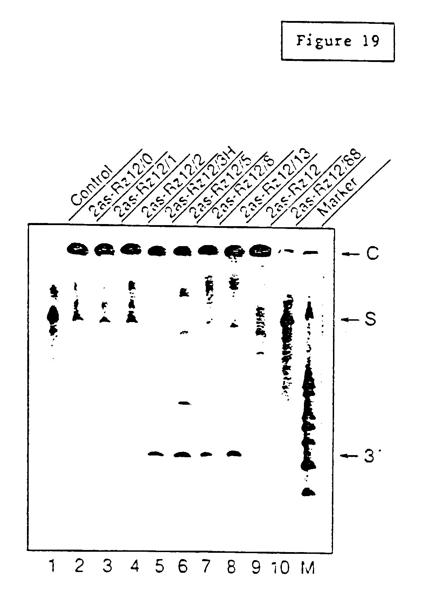

Five additional PCR reactions were performed, using the same reaction conditions, except 100 pg of pBS-Rz12/3 instead of pBS29-Rz12 and instead of Rz12/5 or Rz12/3, the DNA oligonucleotides Rz12/0: d(CCAAGCTTAAAACTGATGAGG) or Rz12/1: d (CCAAGCTTAAACCTGATGAG) or Rz12/2: d(CCAAGCTTAATCCTGATGAG) or Rz12/8: d(GGAAGCTTATTTGTCCTGATGAGG) or Rz12/13: d(GGAAGCTTAGTATTTGTCCTGATGAGG), respectively. The resulting PCR products were also cleaved with SacI and HindIII and cloned into pT3T71ac, yielding the plasmids pBS-Rz12/0, pBS-Rz12/1, pBS-Rz12/2, pBS-Rz12/8 and pBS-Rz12/13, respectively. The relevant sequence was confirmed by sequencing. For generation of plasmid pBS-Rz12/88 the two DNA oligonucleotides SX1: d(CAAGGCTGTTTCGGCC) and SX2: d(TCGAGGCCGAAACAGCCTTGAGCT) where phosphorylated at the 5'-terminus with the aid of T4 kinase, annealed to each other and ligated into the plasmid pBS-Rz12/8 that had been cleaved with SacI and XhoI. The correct construction was confirmed by sequencing.
The relevant sequences of the DNAs are shown in Figure 4 and the resulting RNA transcripts obtained by T3 RNA polymerase from all these plasmids is depicted in Figure 5, where the conformation of the resulting asymmetric hammerhead ribozyme RNAs is indicated.
A ribozyme assay is given in Figures 18 and 19, and the cleavage rates of some constructs are summarized in Table II.

**Table II.**

| Cleavage rate constants of different catalytic antisense RNAs. | | |
|---|---|---|
| RNA | Helix I | *k*_{cleav}^{a} |
| | base pairs | s⁻¹ |
| 2as-Rz12/1 | 1 | 6.0 x 10⁻⁶ |
| 2as-Rz12/2 | 2 | 1.1 x 10⁻⁵ |
| 2as-Rz12/3 | 3 | 1.7 x 10⁻⁴ |
| 2as-Rz12/5 | 5 | 1.7 x 10⁻⁴ |
| 2as-Rz12 | 128 | 2.1 x 10⁻⁴ |

| | | |
|---|---|---|
| ^{a}The standard deviation was 10% or less; | | |

### Example 2: Generation of an asymmetric hammerhead ribozyme with the aid of PCR

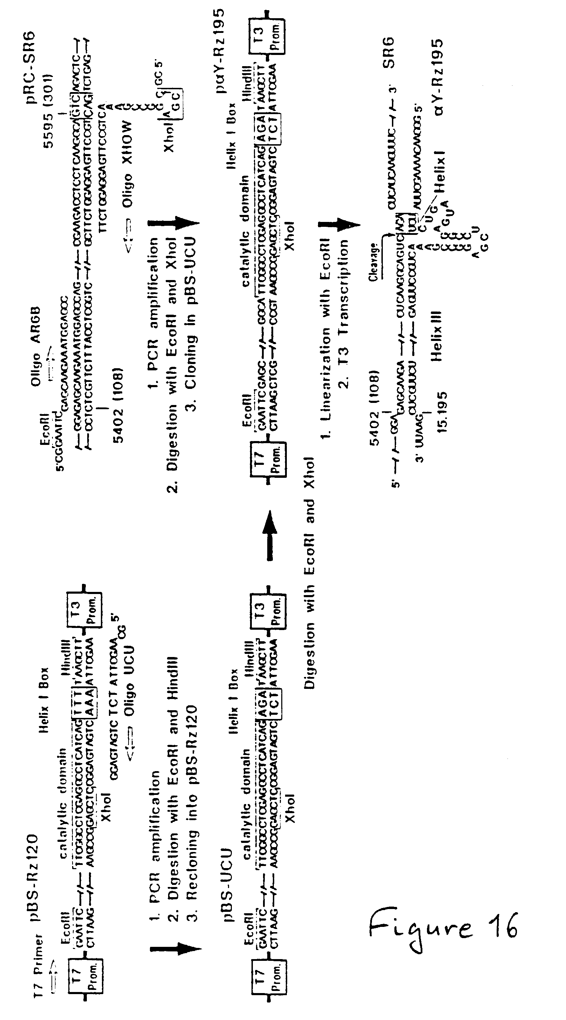

In order to generate a second HIVl-directed asymmetric hammerhead ribozyme the plasmid pAR6 (Sczakiel et al., J.Virol 66 (1992), 5576-5581) was selected (Figure 7A). A particular GUC sequence was selected as cleavable motif (Figure 7A and 16), which in this case is followed by a downstream AGA motif within the target RNA. An asymmetric hammerhead ribozyme that is able to cleave this motif requires an UCU sequence that can form a Helix I with the target RNA. In order to introduce the catalytic domain a PCR reaction was carried out, using the same experimental conditions as in experiment 1, but DNA of pAR6 and the two DNA oligonucleotides AR6B and XHOW. Their sequence is given in Figure 7B and Figure 7C, as well as in Figure 16 which show their binding sites to pAR6 DNA. The PCR product (Figures 7D and 16) does neither contain the complete sequence of the catalytic domain of a hammerhead ribozyme, nor its Helix I region.
The plasmid pBS-Rz12/0 does not encode a catalytically active asymmetric hammerhead ribozyme, since it cannot form a Helix I with its target RNA (compare Figure 5). It might, however, be used as basis for construction of an asymmetric hammerhead ribozyme that forms a three nucleotide Helix I consisting of AAA for the nucleotides 2.3, 2.2, 2.1, since these nucleotides are neighboring the sequence of the catalytic domain (compare Figure 4). The technique for such a construction is described in more detail below (see also Figures 8, 9).
The plasmid pBS-Rz12/0 can also be used for generating any other of the further 63 conceivable trinucleotide sequences different to AAA. This nucleotide sequence, representing the nucleotides 2.3, 2.2, 2.1 of a hammerhead ribozyme will be called "Helix I box" in the following. In Figure 8A-C the conversion of a AAA Box into TCT box is described. For this purpose the DNA oligonucleotide UCU was synthesized (Figure 8A), having the sequence : d(GCAAGCTTATCTCTGATGAGG). This DNA oligonucleotide was used together with plasmid DNA of pBS-Rz12/0 and the T7 Primer DNA oligonucleotide (compare experiment 1) in an analog PCR reaction as outlined under Example 1. The PCR product was cleaved with SacI and HindIII and cloned into pT3T71ac, yielding plasmid pBS-UCU (Figure 8C), which has a Helix I box: TCT. The correct manipulation was confirmed by sequencing.
Similarly, like the AAA Box of pBS-Rz12/0 has been converted to a TCT box it has been into 5 alternative Helix I boxes and it can be converted into any of the conceivable three-nucleotide sequences.

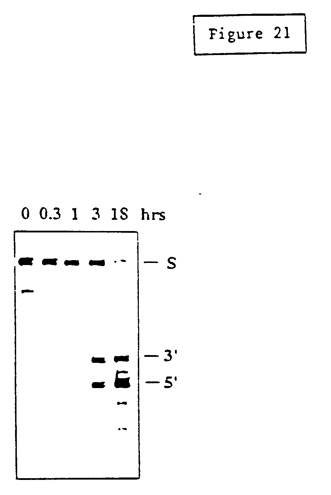

The actually desired asymmetric hammerhead ribozyme was then constructed by recombining pBS-UCU with the PCR fragment obtained with pAR6 and the DNA oligos AR6B and XHOW. Both DNAs where each cleaved with EcoRI and XhoI (Figure 8D,E) and the EcoRI-XhoI fragment of the PCR product was inserted into the pBS-UCU cut with EcoRI and XhoI, yielding plasmid pαY-Rz195 (Figure 8F). After transcription with T3 RNA polymerase the RNA αY-Rz195 was synthesized. The RNA can form with its target RNA the asymmetric hammerhead ribozyme with a three nucleotide Helix I region (Figure 8G).
Figure 8H schematically outlines the origin of the DNAs used for construction of pαY-Rz195. The PCR product delivers the Helix III forming region and a part of Helix II including the nucleotides 12, 13, 14 (compare Figure 2) and the Loop 2 nucleotides. The other part comes form the unrelated plasmid pBS-UCU which delivers the entire Helix I region, the nucleotides 3-9 of the catalytic domain, plus the nucleotides 10.1 till 10.4 which form the 5' half of Helix II and the Loop 2 nucleotides. Recombination of the two DNAs occurred via the XhoI site C/TCGAG, which is present in both DNAs. Figure 8I indicates the nucleotide numbers of the part relevant for ribozyme construction that is present in the pBS-UCU vector and that corresponds to the general formula of claim 1.
In parallel to the plasmid pαY-Rz195 a second plasmid pαY-Rzl95inac was constructed with the DNA oligonucleotide XHOM which differs from XHOW in one nucleotide. The resulting asymmetric hammerhead ribozyme is lacking the nucleotide 12 of the catalytic domain (a G nucleotide) and is therefore completely inactive. A ribozyme assay of αY195 is given in Figure 21.

### Example 3: Generation of a set of plasmid vectors having a three nucleotide Helix I Box.

In principle any desired three nucleotide Helix I Box can be generated by manipulating plasmid pBS-12/0 or related plasmids which can then be combined with a PCR product that delivers the complementing part of the catalytic domain of a hammerhead ribozyme plus the Helix III forming region as described in Example 2 and Figure 8. In order to make the PCR amplification more economical it would be desirable to shorten the sequence that overlaps with the catalytic domain of a hammerhead ribozyme and to include a greater part of the sequence of the catalytic domain into the vector. The strategy for this is outlined in Figure 9 and in Figure 17. The vector in Figure 9 provides nucleotides 2.3, 2.2, 2.1, which correspond to the Helix I Box, and the nucleotides 3 till 12 of the catalytic domain (Figure 9A). The PCR product provides the nucleotides 11.1, 12, 13, 14 of the catalytic domain and the Helix III forming nucleotides 15.1 till 15.y. Recombination occurs via nucleotides 11.1 and 12. This general strategy is achieved by creating vectors as given in Figure 9B. The plasmid consists of two restriction sites A and B, wherein A and B can be any reasonable restriction site, each site A and B can also consist of several restriction recognition sequences (polylinker region) and at least B is also dispensable. The sequence encoding the part of the catalytic domain of a hammerhead ribozyme as given in Figure 9A is contained between an AccI site of the sequence GT/CGAC and the Helix I Box. Upstream of the Helix I Box is an appropriate promoter (Figure 9B). The PCR product (Figure 9C) should contain a restriction site A and a terminal SfuI site. By recombining the DNA fragment cleaved with restriction enzyme A and AccI (vector) and restriction enzyme A and SfuI (PCR product) (Figure 9D) the recombinant DNA (Figure 9E) is created from which the asymmetric hammerhead ribozyme as given in Figure 9F can be synthesized.
Figure 10 shows a specific example of such a vector plasmid (pIMBB series) that can be used for simple generation of asymmetric hammerhead ribozymes.
It is based on the plasmid pT3T71ac which contains the sequence between the AccI and SphI sites as indicated. For construction of plasmids of the pIMBB-series, the two DNA oligonucleotides Pat3a and Pat3b were synthesized (Figure 10C). Both DNA oligonucleotides contain a randomized three nucleotide sequence. By inserting the annealed and phosphorylated DNA oligonucleotides into the pT3T71ac a set of 64 different plasmids is obtained. Sequencing of individual plasmid clones identifies a particular Helix I Box. Specific boxes can be made by synthesizing a specific pair of DNA oligonucleotides.
Based on the general sequence shown in Figure 10, the following plasmids have been generated and used for ribozyme construction with varying substrates: pIMBB-GCT/AGC, pIMBB-GTT/AAC, pIMBB-AGG/TCC, wherein GCT, GTT and AGG represent the corresponding Helix I boxes.
A similar strategy as outlined in Figure 9 is given and described in Figure 17.
Here the cloning site used for inserting the PCR fragment into one of the vectors of the pFORTH series is EagI, which becomes regenerated during cloning. Similar vector systems are conceivable.
Also the entire catalytic domain plus the Helix I-forming region can be delivered by a DNA oligonucleotide that will be used for PCR amplification.

### Example 4: Generation of plasmid vectors having a four-nucleotide Helix I Box.

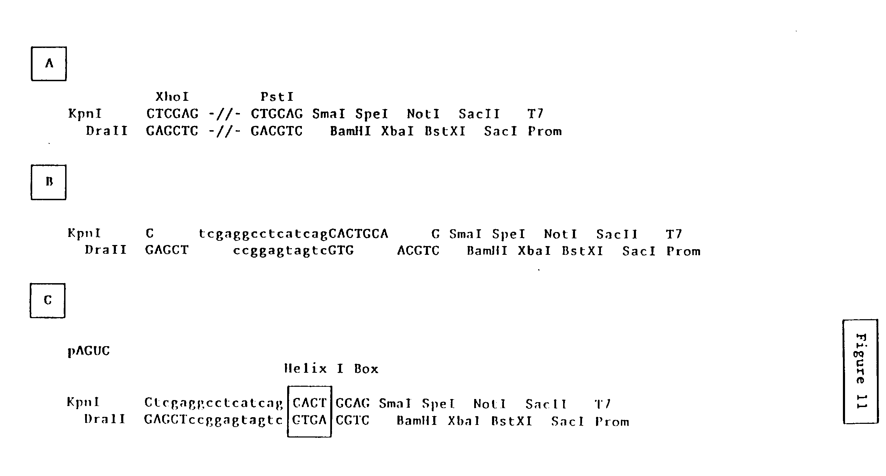

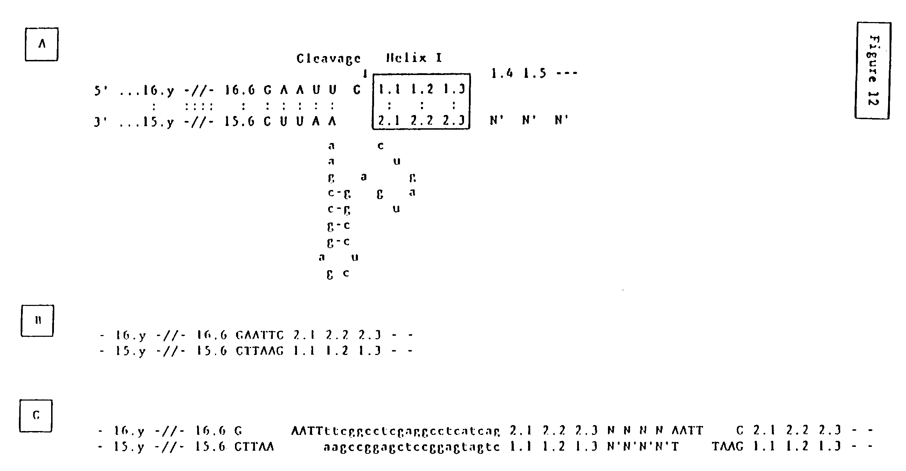

In order to generate a vector with a Helix I Box longer than three nucleotides and suitable for the PCR strategy to generate asymmetric hammerhead ribozymes as outlined in Figures 8 and 9 a specific DNA cassette can be inserted. The Figure 11 shows the insertion of a synthetic DNA cassette consisting of the annealed and phosphorylated DNA oligonucleotides HB1 and HB2 into the plasmid pBluescript II KS (Stratagene) in order to generate a four nucleotide Helix I Box consisting of nucleotides AGTG. The correct sequence manipulations in the resulting plasmid pAGUG were confirmed by sequencing.

### Example 5: Generation of asymmetric hammerhead ribozymes by insertion of a synthetic cDNA cassette into restriction recognition sites.

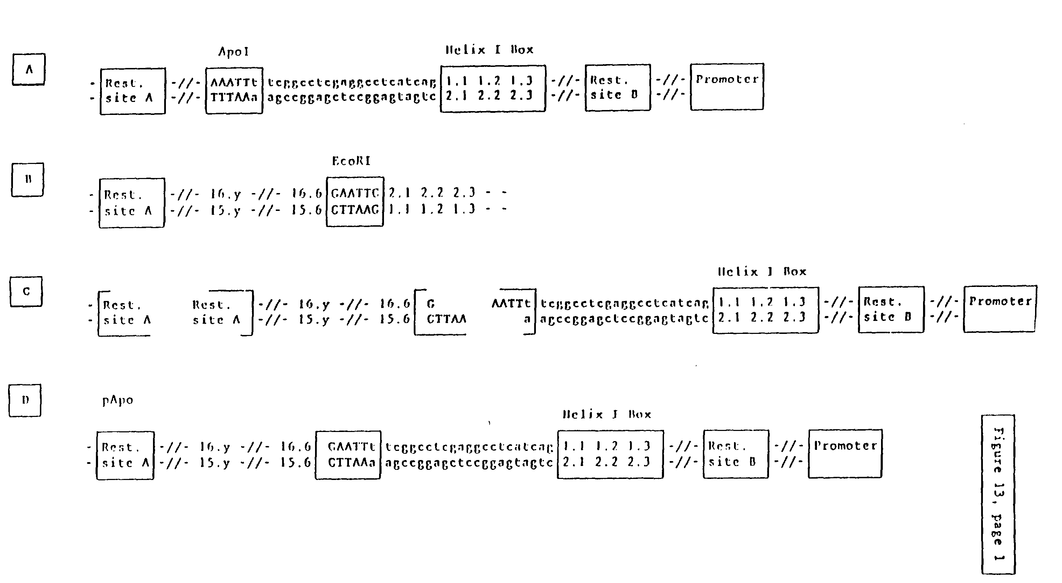

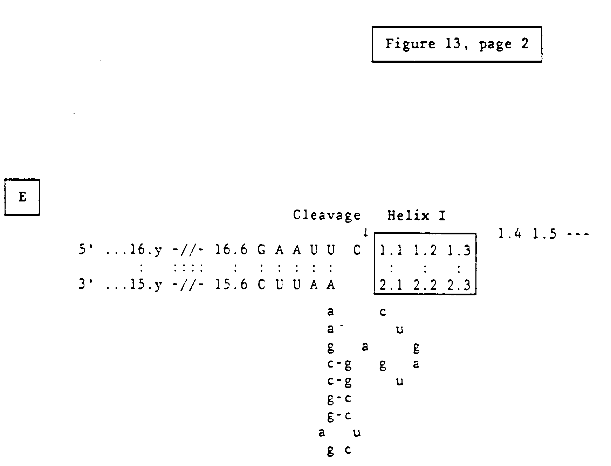

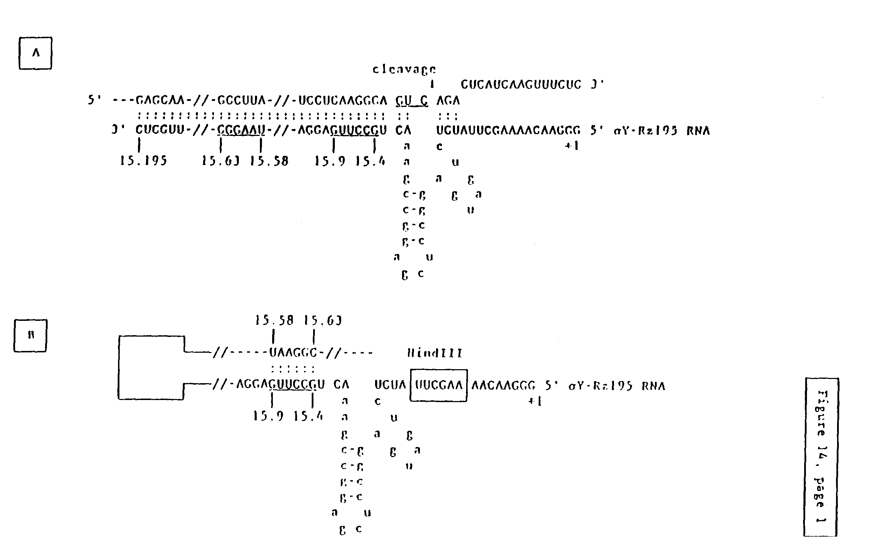

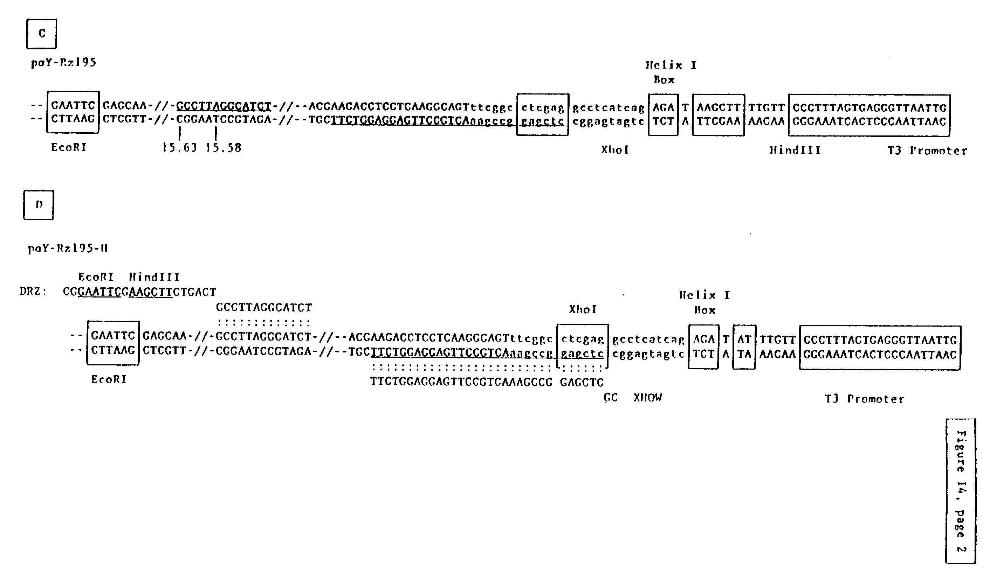

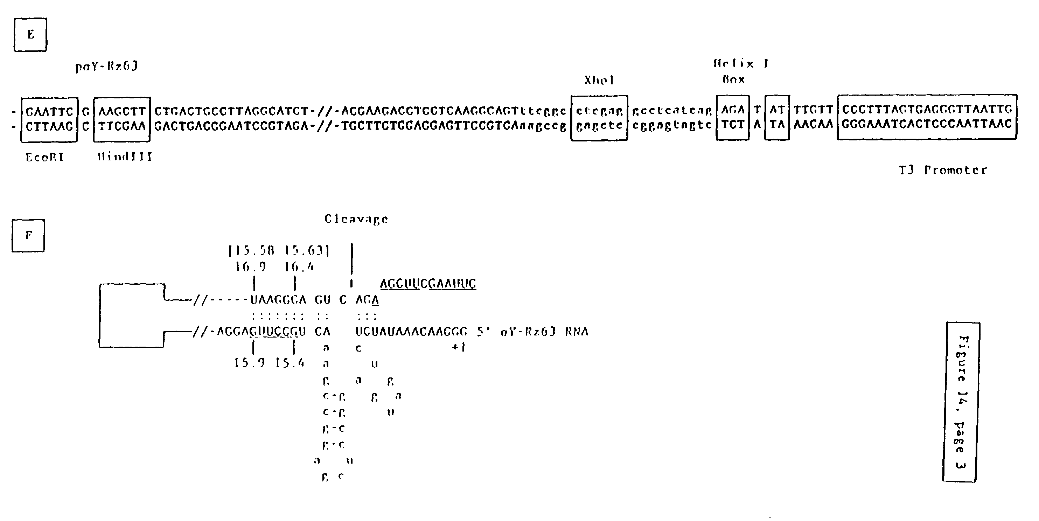

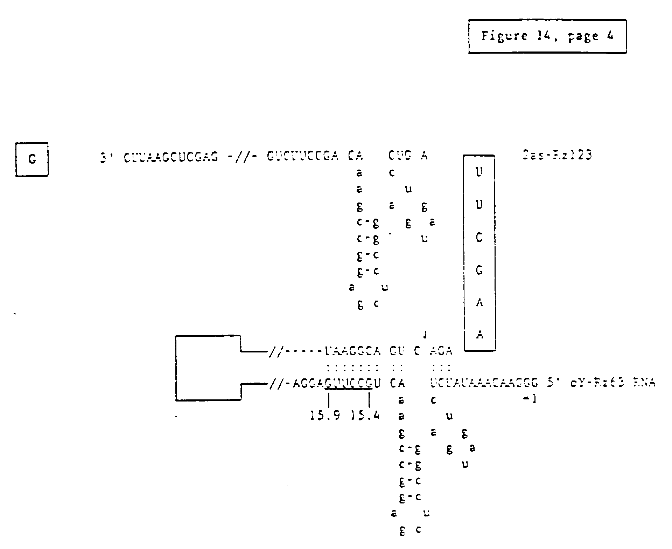

Some restriction recognition sequences contain a cleavable motif and can be used for the insertion of synthetic DNA cassettes to generate a DNA encoding an asymmetric hammerhead ribozyme. In particular useful are those restriction recognition sequences in which the potential nucleotide 17 of the cleavable motif of the target RNA is part of the restriction recognition sequence but not part of the protruding ends created after digestion with said restriction enzyme. For example EcoRI (G/AATTC) contains the UUC motif, ApoI (R/AATTY) contains either an UUC or UUU motif, Psp1406I (AA/CGTT) contains a GUU motif, AatI (GACGT/C) and SnaBI (TAC/GTC) contain a GUC motif, HindIII (A/AGCTT) contains a CUU motif, Ecl126II (GAG/CTC) and SacI (GAGCT/C) contain a CUC motif, SspI (ATA/TAT) contains an AUA motif and EcoRV (GAT/ATC) contains an AUC motif. Any RNA sequence that contains any of these restriction recognition sequences can be targeted by an asymmetric hammerhead ribozyme as shown for the EcoRI restriction recognition sequence in Figure 12A. The corresponding double-stranded cDNA that encodes this target RNA is shown in Figure 12B. After digestion with EcoRI, the synthetic DNA cassette shown in the middle part of Figure 12C can be inserted, generating a DNA encoding an asymmetric hammerhead ribozyme. The nucleotide sequences illustrated with N and N' may contain other useful sequences such as restriction recognition sequences and/or promoter sequences. Similar strategies can be developed for each of the above mentioned and further suitable restriction recognition sequences. This procedure of generating an asymmetric hammerhead ribozyme does not require the usage of a PCR reaction.
The procedure can be modified as outlined in Figure 13. Here the sequence corresponding to the synthetic DNA cassette is provided by a DNA vector of the pApo-series, consisting of one or several restriction recognition sites A followed by a restriction recognition sequence for ApoI, the sequence encoding the catalytic domain and a Helix I Box (64 combinations possible), followed optionally by one or several restriction recognition sites B and an optional promoter (Figure 13A). The cDNA which encodes the target RNA and contains a restriction recognition sequence for EcoRI (Figure 13B) and any of the restriction recognition sequences that are present in the vector of Figure 13A is suitable for conversion into a DNA encoding for a asymmetric hammerhead ribozyme directed against the UUC motif contained in the EcoRI restriction recognition sequence. For this purpose the cDNA is digested with EcoRI and an appropriate restriction enzyme A. The vector is digested with ApoI and the appropriate restriction enzyme A and both fragments are ligated (Figure 13C) resulting in the recombinant DNA (Figure 13D) which encodes the asymmetric hammerhead ribozyme shown in Figure 13E. Also this procedure does not require a PCR step.

### Example 6: Generation of multiple asymmetric hammerhead ribozymes.

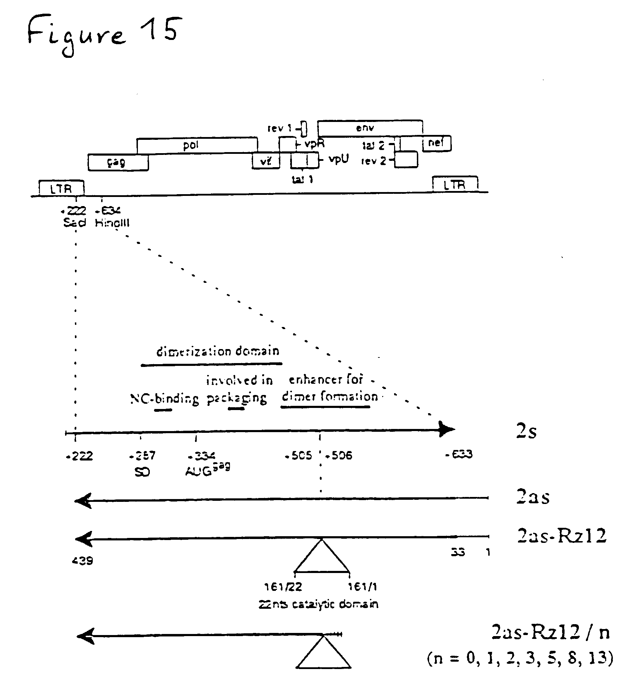

As mentioned before, asymmetric hammerhead ribozymes are characterized by the shortness of the Helix I-forming region. Therefore it is also possible to engineer a "sense box" at the 3'-terminal region of the asymmetric hammerhead ribozyme RNA in order to allow formation of an intramolecular hammerhead complex. In the αY-Rz195 RNA there is a hexanucleotide sequence, corresponding to nucleotides 15.58 till 15.63 according to the conventional numbering system for hammerhead ribozymes (Hertel et al., supra) (Figure 14A and Figure 7A), which is complementary to nucleotides 15.4 till 15.9 (Figure 14A). In principle, these two sequences could interact and form a secondary structure given in Figure 14B. The RNA αY-Rz195 is encoded by the plasmid pαY-Rz195 (Figure 14C and Figure 8F). By converting this DNA into a DNA that encodes for an asymmetric hammerhead ribozyme that is able to self-cleave intramolecularly it is possible to attach a second asymmetric hammerhead ribozyme downstream of the cleavage site in order to create a "tandem" ribozyme. For this purpose plasmid pαY-Rz195 was first cleaved with HindIII followed by treatment with nuclease S1 and re-ligation which destroyed its HindIII restriction recognition sequence. The resulting plasmid pαY-Rz195-H is given in Figure 14D. From this plasmid a DNA fragment was amplified by PCR, using the two DNA oligonucleotides DRZ (Figure 14D) and XHOW (Figures 14D and 7B) under the same experimental conditions like in Example 1. The resulting PCR product was cleaved with EcoRI and XhoI and re-cloned into the same sites of plasmid pαY-Rz195-H. The resulting recombinant plasmid pαY-Rz63 is given in Figure 14E. The DNA oligonucleotide DRZ newly introduced a HindIII restriction recognition sequence (Figure 14D and E). The resulting RNA transcript αY-Rz63 is able to form an intramolecular hammerhead complex (Figure 14F), which itself is an asymmetric hammerhead ribozyme.
The plasmid pαY-Rz63 has two restriction recognition sequences: EcoRI and HindIII which allow the insertion of a "downstream" ribozyme sequence. This could, for example, be the EcoRI - HindIII fragment of plasmid pαY-Rz195 (Figure 14C). The resulting RNA transcript would contain two asymmetric hammerhead ribozyme molecules that could cleave the target RNA shown in Figure 14A. These RNAs are physically linked during transcription but can self-release due to the formation of the intramolecular hammerhead conformation.
The second RNA can, however, be completely unrelated. For example the EcoRI-HindIII fragment of plasmid pBS-Rz12/3 (Figure 4, the EcoRI site is not detailed there, but it directly neighbors the SacI site) was cloned into plasmid pαY-Rz63. The resulting RNA transcript (Figure 14G) consists of two domains, the 5' located sequence corresponding to αY-Rz63 and, connected via an AAGCUU sequence, representing the HindIII restriction recognition sequence, the 3' located sequence corresponding to 2as-Rz123 (Figure 5). Since the αY-Rz63 domain is able to form an intramolecular complex of an asymmetric hammerhead ribozyme, the two RNAs shown in Figure 15H will be released.

In principle, an unlimited number of asymmetric hammerhead ribozymes could be joined to multiple ribozyme sequences that self-excise after transcription. This is particularly important when a target RNA, for example a viral RNA, should be targeted by a ribozyme simultaneously at different sites. This can be easily achieved by providing a single RNA transcript that self-excises into the individual asymmetric hammerhead ribozymes. It should be stressed that it is not required to find a nucleotide sequence within the Helix III forming part that has some sense polarity as it was the case for αY-Rz195. Such a "sense box" which can provide an intramolecular Helix III- and Helix I-region including the cleavable motif can be engineered completely artificially.

### Example 7: Determination of cleavage rates for asymmetric hammerhead ribozymes

Radioactively labelled substrate RNA was obtained by transcription with T7 RNA polymerase from HindIII-linearized plasmid pBS29-CX as described by Homann et al., Nucleic Acids Res., **21** (1993), 2809-2814, or from NotI-linearized plasmid pRC-SR6 described by Homann et al., J. Mol. Biol., **223** (1993), 7-15, which contains the relevant sequence of pAR6 suitable for transcription.
For generation of ribozyme RNA, the plasmids pBS29-Rz12 and pBS-Rz12/88 were linearized with SacI followed by trimming with T4 DNA polymerase, all other ribozyme-encoding plasmids with EcoRI. All ribozymes were synthesized with T3 RNA polymerase as described by Homann et al., Nucleic Acids Res., **21** (1993), 2809-2814.

The association rate constant of all ribozyme RNAs, except transcript 2as-Rz12/88, to its target were found to be in the same range as that of 2as-Rz12 which had been determined earlier to be 1.5 x 10⁵ M⁻¹s⁻¹ by Homann et al., Nucleic Acids Res., **21** (1993), 2809-2814.

All ribozyme assays were carried out under conditions for single turnover. Ribozyme RNAs were used in a final concentration of about 200 nM and mixed with radioactively labelled substrate RNA (final concentration about 2 nM) in a reaction volume of 5 µl, containing 20 mM MgCl₂ and 50 mM Tris/HCl pH 8.0. Under these reaction conditions, the substrate RNA becomes quickly associated with ribozyme RNA at similar rates for all constructs. For example, at kₐₛₛ≈ 1.5 x 10 M⁻¹s⁻¹ and 200 nM of the rate limiting RNA concentration, the half life for association was in the order of half a minute. The reaction was stopped by adding EDTA to a final concentration of 25 mM, followed by addition of 1 µg tRNA and ethanol precipitation. The reaction products were separated on a 5% polyacrylamide gel containing 8M urea as described by Tsagris et al, EMBO J., (1987) **8**, 2173-2183. For separation on a native gel, the urea was omitted and samples were loaded without dissolving them in formamide and without prior boiling. After drying the gels, RNAs were visualized by autoradiography.
Figure 18 shows the results of the cleavage assay carried out with the set of 5'-truncated 2as-Rzl2-derived RNAs. If the Helix I-forming region of the catalytic RNA was three nucleotides or longer, an uniform cleavage efficiency could be detected at any reaction time. For example, the RNA 2as-Rz12/3 which can form a Helix I of only three nucleotides (compare Figure 5) is equally active as 2as-Rz12 that forms a Helix I of 128 base pairs (compare
Figure 18A,B, lanes 5 and 9). Also all other RNAs with Helices I of 5-13 nucleotides in length had the same cleavage efficiency (for rate constants see Table II; compare also Figure 18A,B, lanes 6-8). The ribozyme 2as-Rz12/2, having a two base Helix I-forming region (Figure 5) delivered some cleavage products that became detectable after a reaction time of 60 minutes (Figure 18B, lane 4), the cleavage rate was, however, about 15-fold reduced (Table II). Traces of cleaved substrate RNA could even be detected on the original autoradiographs for ribozyme 2as-Rz12/1 (Figure 5) and after longer reaction times. The ribozyme 2as-Rz12/0 (Figure 5), which entirely lacks a matching helix I-forming region did not generate specific cleavage products.
When the ribozyme 2as-Rz12/88 (which has flanking regions of 8 nucleotides on either side, Figure 5) was tested under the same reaction conditions as the 5'-terminally truncated 2as-Rz-derived RNAs, it did not generate any specific cleavage (Figure 18A,B, lane 10). In order to ensure that the reaction temperature was below the melting temperature for duplex RNA, the reaction was repeated at 20°C, but again neither cleavage was observed (data not shown). However, if the mixture of the substrate RNA and the ribozyme RNA 2as-Rz12/88 was heated for 5 minutes at 60°C, followed by fast chilling in ice and subsequent incubation at 37°C for 60 minutes, it became evident that this ribozyme can cleave the substrate as well as the other 2as-Rz12 derivatives (Figure 18B, lane 11). This finding indicates that without prior heat treatment, the ribozyme 2as-Rz12/88 (with an "8 plus 8 design") could not associate properly with its target RNA 2as.
When the reaction products of a ribozyme reaction were separated on a native polyacrylamide gel (Figure 19), it was observed that asymmetric catalytic RNAs released the 3'-terminal cleavage product, whereas the parental catalytic antisense RNA 2as-Rz12 remained bound to the cleaved substrate RNA. Further, it was confirmed that the substrate RNA could not complex with the 2as-Rz12/88 RNA.

### Example 8: Inhibition of HIV-1 replication by asymmetric hammerhead ribozymes

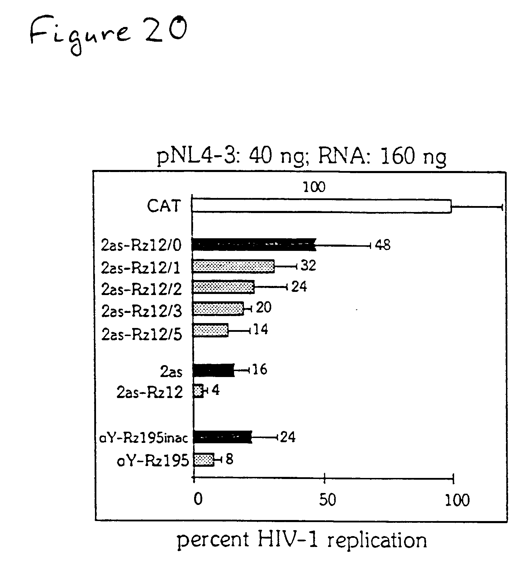

For measurements of the effects of asymmetric hammerhead ribozymes on HIV-1 replication, in *vitro* synthesized RNAs at amounts of 160 ng per well and infectious proviral HIV-1 DNA pNL4-3 at 40 ng, respectively, were co-transfected into SW480 cells grown semi-confluent in 48 well plates by calciumphosphate co-precipitation essentially as described by Rittner et al., Nucleic Acids Res., **21** (1993), 1381-1387. One day after transfection, 1 x 10⁵ MT-4 cells were added and the final volume was adjusted to 1 ml with RPMI 1640 medium. Virus replication was measured 5 days after transfection with dilutions of cell-free culture supernatants by a commercial HIV-1 antigen ELISA (Organon, Holland). The data of the inhibition assay are summarized in Figure 20.

## Claims

1. A nucleotide sequence comprising the following general formula
3'-[N]₁₁-[N]_{Lo}-[N]₁₀-[N]₉₋₃-[N]-5'
wherein N represents any nucleotide and may be the same or different, [N] represents no nucleotide, nucleotide 2.1 or nucleotides 2.x till 2.1 capable of forming Helix I in a hammerhead ribozyme/target-complex, [N]₉₋₃ represents nucleotides 3 to 9 of a hammerhead ribozyme, [N]₁₀ represents nucleotides 10.1 till 10.n and [N]₁₁ represents no nucleotide, nucleotide 11.m or comprises at least a part of nucleotides 11.m till 11.1, x is an integer ranging from 2 to 8, m is an integer, n is an integer and represents the length of Helix II, wherein, if [N]₁₁ comprises at least a part of nucleotides 11.m till 11.1, n and m may be the same or different, Lo is an integer and [N]_{Lo} represents any sequence capable of forming Loop 2 of a hammerhead ribozyme, wherein said nucleotide sequence does not contain the Helix III-forming region of a hammerhead ribozyme/target-complex.

2. The nucleotide sequence according to claim 1, wherein the region 3'-[N]₁₁-[N]_{Lo}-[N]₁₀-5' contains at least one recognition. sequence for one or more restriction enzymes and/or a recognition sequence for a restriction enzyme that overlaps with the sequence following 3' to [N]₁₁.

3. The nucleotide sequence according to claim 1 or 2, wherein n ranges from 3 to 50.

4. The nucleotide sequence according to anyone of claims 1 to 3, wherein x is 3 and/or n is 4 and/or m ranges from 2 to 4.

5. A nucleic acid comprising the nucleotide sequence of anyone of claims 1 to 4 and a second nucleotide sequence containing at least the nucleotides 12 to 14 of a hammerhead ribozyme and at least part of the Helix III-forming region of a hammerhead ribozyme/target-complex, starting with nucleotide 15.1 till 15.y, wherein y is an integer and the number of nucleotides within the Helix I-forming part, corresponding to nucleotides 2.1 till 2.x, is smaller than the number of nucleotides of the Helix III-forming region, corresponding to nucleotides 15.1 till 15.y.

6. The nucleic acid according to claim 5, wherein the nucleotide sequence and the second nucleotide sequence are linked via one of the recognition sequences contained in 3'-[N]₁₁-[N]_{Lo}-[N]₁₀-5' or via a recognition sequence for a restriction enzyme that overlaps with the sequence following 3' to [N]₁₁.

7. The nucleic acid according to claim 5 or 6, further comprising a third nucleotide sequence adjacent to the Helix III-forming region of the second nucleotide sequence, containing at least 3 nucleotides capable of forming an intramolecular Helix III of a hammerhead ribozyme, followed directly by nucleotide 17 of a hammerhead ribozyme and a nucleotide sequence which is complementary to [N].

8. An asymmetric hammerhead ribozyme comprising the nucleic acid of anyone of claims 5 to 7 or the nucleotide sequence of anyone of claims 1 to 4.

9. The asymmetric hammerhead ribozyme according to claim 8, wherein x ranges from 2 to 8.

10. An asymmetric hammerhead ribozyme, wherein the Helix I-forming region is smaller than the Helix III-forming region.

11. The asymmetric hammerhead ribozyme according to anyone of claims 8 to 10, wherein at least one nucleotide is chemically modified.

12. The asymmetric hammerhead ribozyme according to anyone of claims 8 to 11, which is chemically synthesized.

13. An RNA construct comprising at least two asymmetric hammerhead ribozymes of anyone of claims 8 to 12.

14. The RNA construct according to claim 13, wherein the Helix I- and Helix III-forming regions of the asymmetric hammerhead ribozymes may be the same or different.

15. A DNA sequence which upon transcription corresponds to the asymmetric hammerhead ribozyme of anyone of claims 8 to 12 or the RNA construct of claim 13 or 14.

16. A vector comprising the nucleotide sequence of anyone of claims 1 to 4, the nucleic acid of anyone of claims 5 to 7, the asymmetric hammerhead ribozyme of anyone of claims 8 to 12, the RNA construct of claim 13 or 14 or the DNA sequence of claim 15.

17. A host organism containing the nucleotide sequence of anyone of claims 1 to 4, the nucleic acid of anyone of claims 5 to 7, the asymmetric hammerhead ribozyme of anyone of claims 8 to 12, the RNA construct of claim 13 or 14, the DNA sequence of claim 15 or the vector of claim 16.

18. A method for the production of the nucleotide sequence of anyone of claims 1 to 4, the nucleic acid of anyone of claims 5 to 7, the asymmetric hammerhead ribozyme of anyone of claims 8 to 12, the RNA construct of claim 13 or 14, the DNA sequence of claim 15 or the vector of claim 16, comprising cultivating the host organism of claim 17 under suitable conditions and isolating the desired product from the culture.

19. A process for the preparation of the asymmetric hammerhead ribozyme of anyone of claims 8 to 12, the RNA construct of claim 13 or 14, or the DNA encoding said asymmetric hammerhead ribozyme or said RNA construct, comprising
(i) selecting a cleavable motif in the target RNA,
(ii) selecting the appropriate vector and/or DNA cassette that provides a Helix I Box matching to the selected cleavable motif and a part of the catalytic domain of a hammerhead ribozyme as defined by the general formula 3'-[N]₁₁-[N]_{Lo}-[N]₁₀-[N]₉₋₃-[N]-5' and
(iii) attaching the second sequence that corresponds to the Helix III-forming part and the residual part of the catalytic domain of a hammerhead ribozyme.

20. A process for the preparation of the asymmetric hammerhead ribozyme of anyone of claims 8 to 12, the RNA construct of claim 13 or 14, or the DNA encoding said asymmetric hammerhead ribozyme or said RNA construct, comprising introducing the catalytic domain and the Helix I-forming sequence into DNA by a single PCR step, in which one DNA oligonucleotide provides the sequence of at least part of the catalytic domain and the Helix I-forming region, or the step of the chemical synthesis of at least a part of the asymmetric ribozymes, or the generation of multiple ribozymes by adding a short sense sequence forming an intramolecular Helix III- and Helix I-forming region, including a cleavable motif.

21. A composition containing the asymmetric hammerhead ribozyme of anyone of claims 8 to 12, the RNA construct of claim 13 or 14, the DNA sequence of claim 15 encoding said asymmetric hammerhead ribozyme or said RNA construct or the vector of claim 16 containing said asymmetric hammerhead ribozyme, said RNA construct or said DNA sequence, optionally in association with a pharmaceutically, veterinarian or agriculturally acceptable carrier and/or diluent.

## Patentansprüche

1. Nukleotidsequenz, umfassend die folgende allgemeine Formel
3'-[N]₁₁-[N]_{Lo}-[N]₁₀-[N]₉₋₃-[N]-5'
worin N ein beliebiges Nukleotid bedeutet und gleich oder unterschiedlich sein kann, [N] kein Nukleotid, Nukleotid 2.1 oder Nukleotide 2.x bis 2.1, befähigt zur Bildung einer Helix I in einem Hammerhead-Ribozym/Target-Komplex bedeutet, [N]₉₋₃ Nukleotide 3 bis 9 eines Hammerhead-Ribozyms bedeutet, [N]₁₀ Nukleotide 10.1 bis 10.n bedeutet und [N]₁₁ kein Nukleotid, Nukleotid 11.m bedeutet oder mindestens ein Teil der Nukleotide 11.m bis 11.1 umfaßt, x eine ganze Zahl im Bereich von 2 bis 8 ist, m eine ganze Zahl ist, n eine ganze Zahl ist und die Länge von Helix II bedeutet, worin, wenn [N]₁₁ mindestens ein Teil der Nukleotide 11.m bis 11.1 umfaßt, n und m gleich oder unterschiedlich sein können, Lo eine ganze Zahl ist und [N]_{Lo} irgendeine Sequenz bedeutet, die befähigt ist, einen Loop 2 eines Hammerhead-Ribozyms zu bilden, wobei die Nukleotid-Sequenz keine Helix III-bildende Region eines Hammerhead-Ribozym/Target-Komplexes enthält.

2. Nukleotidsequenz nach Anspruch 1, worin die Region 3'-[N]₁₁-[N]_{Lo}-[N]₁₀-5' mindestens eine Erkennungssequenz für ein oder mehrere Restriktionsenzyme enthält und/oder eine Erkennungssequenz für ein Restriktionsenzym, die mit der Sequenz, welche 3' von [N]₁₁ folgt, überlappt.

3. Nukleotidsequenz nach Anspruch 1 oder 2, worin n im Bereich von 3 bis 50 liegt.

4. Nukleotidsequenz nach einem der Ansprüche 1 bis 3, worin x 3 ist und/oder n 4 ist und/oder m im Bereich von 2 bis 4 liegt.

5. Nukleinsäure, umfassend die Nukleotidsequenz nach einem der Ansprüche 1 bis 4 und eine zweite Nukleotidsequenz, welche mindestens die Nukleotide 12 bis 14 eines Hammerhead-Ribozyms enthält und mindestens ein Teil der Helix III-bildenden Region eines Hammerhead-Ribozyms/Target-Komplexes, beginnend mit den Nukleotiden 15.1 bis 15.y, worin y eine ganze Zahl ist und die Anzahl der Nukleotide im Helix 1-bildenden Teil, entsprechend den Nukleotiden 2.1 bis 2.x, kleiner ist als die Anzahl der Nukleotide der Helix III-bildenden Region, entsprechend den Nukleotiden 15.1 bis 15.y.

6. Nukleinsäure nach Anspruch 5, worin die Nukleotidsequenz und die zweite Nukleotidsequenz verknüpft sind über eine der Erkennungssequenzen, die in der Sequenz 3'-[N]₁₁-[N]_{Lo}-[N]₁₀-5' enthalten ist, oder über eine Erkennungssequenz für ein Restriktionsenzym, die mit einer Sequenz, welche 3' von [N]₁₁ folgt, überlappt.

7. Nukleinsäure nach Anspruch 5 oder 6, welche weiterhin eine dritte Nukleotidsequenz umfaßt, welche benachbart zur Helix III-bildenden Region der zweiten Nukleotidsequenz ist, mindestens 3 Nukleotide, die befähigt sind, eine intramolekulare Helix III eines Hammerhead-Ribozyms auszubilden, welche direkt an Nukleotid 17 eines Hammerhead-Ribozyms anschließt, und eine Nukleotidsequenz, die komplementär zu [N] ist, enthält.

8. Asymmetrisches Hammerhead-Ribozym, umfassend eine Nukleinsäure nach einem der Ansprüche 5 bis 7 oder die Nukleotidsequenz nach einem der Ansprüche 1 bis 4.

9. Asymmetrisches Hammerhead-Ribozym nach Anspruch 8, worin x im Bereich von 2 bis 8 liegt.

10. Asymmetrisches Hammerhead-Ribozym, worin die Helix 1-bildende Region kleiner ist als die Helix III-bildende Region.

11. Asymmetrisches Hammerhead-Ribozym nach einem der Ansprüche 8 bis 10, worin mindestens ein Nukleotid chemisch modifiziert ist.

12. Asymmetrisches Hammerhead-Ribozym nach einem der Ansprüche 8 bis 11, welches chemisch synthetisiert ist.

13. RNA-Konstrukt, umfassend mindestens zwei asymmetrische Hammerhead-Ribozyme nach einem der Ansprüche 8 bis 12.

14. RNA-Konstrukt nach Anspruch 13, worin die Helix I- und Helix III-bildenden Regionen des asymmetrischen Hammerhead-Ribozyms gleich oder unterschiedlich sein können.

15. DNA-Sequenz, welche nach einer Transkription einem asymmetrischen Hammerhead-Ribozym nach einem der Ansprüche 8 bis 12 oder dem RNA-Konstrukt nach Anspruch 13 oder 14 entspricht.

16. Vektor, umfassend die Nukleotidsequenz nach einem der Ansprüche 1 bis 4, die Nukleinsäure nach einem der Ansprüche 5 bis 7, das asymmetrische Hammerhead-Ribozym nach einem der Ansprüche 8 bis 12, das RNA-Konstrukt nach Anspruch 13 oder 14 oder die DNA-Sequenz nach Anspruch 15.

17. Wirtsorganismus, enthaltend die Nukleotidsequenz nach einem der Ansprüche 1 bis 4, die Nukleinsäure nach einem der Ansprüche 5 bis 7, das asymmetrische Hammerhead-Ribozym nach einem der Ansprüche 8 bis 12, das RNA-Konstrukt nach Anspruch 13 oder 14, die DNA-Sequenz nach Anspruch 15 oder den Vektor nach Anspruch 16.

18. Verfahren zur Herstellung der Nukleotidsequenz nach einem der Ansprüche 1 bis 4, der Nukleinsäure nach einem der Ansprüche 5 bis 7, des asymmetrischen Hammerhead-Ribozyms nach einem der Ansprüche 8 bis 12, des RNA-Konstruktes nach Anspruch 13 oder 14, der DNA-Sequenz nach Anspruch 15 oder des Vektors nach Anspruch 16, welches die Züchtung des Wirtsorganismus nach Anspruch 17 unter geeigneten Bedingungen und die Isolierung des gewünschten Produktes von der Kultur umfaßt.

19. Verfahren zur Herstellung eines asymmetrischen Hammerhead-Ribozyms nach einem der Ansprüche 8 bis 12, des RNA-Konstruktes nach Anspruch 13 oder 14 oder der DNA, welche das asymmetrische Hammerhead-Ribozym oder das RNA-Konstrukt kodiert, umfassend
(i) das Selektieren eines spaltbaren Motives in der Target-RNA,
(ii) das Selektieren des geeigneten Vektors und/oder der DNA-Kassette, welche die Helix I Box liefert, die zu dem selektierten Motiv und einem Teil der katalytischen Domäne eines Hammerhead-Ribozyms paßt, wie durch die allgemeine Formel 3'- [N]₁₁ - [N]_{Lo}- [N]₁₀- [N]₉₋₃ - [N] - 5' definiert, und
(iii) das Anfügen der zweiten Sequenz, die dem Helix III-bildenden Teil und den restlichen Teilen der katalytischen Domäne eines Hammerhead-Ribozyms entspricht.

20. Verfahren zur Herstellung eines asymmetrischen Hammerhead-Ribozyms nach einem der Ansprüche 8 bis 12, des RNA-Konstruktes nach Anspruch 13 oder 14 oder der DNA, welche das asymmetrische Hammerhead-Ribozym oder das RNA-Konstrukt kodiert, umfassend das Einführen der katalytischen Domäne und der Helix 1-bildenden Sequenz in DNA durch einen einzigen PCR-Schritt, in dem ein DNA-Oligonukleotid die Sequenz von mindestens einem Teil der katalytischen Domäne und der Helix 1-bildenden Region liefert, oder den Schritt der chemischen Synthese von mindestens einem Teil des asymmetrischen Hammerhead-Ribozymes, oder das Erzeugen von multiplen Ribozymen durch Hinzufügen einer kurzen sense-Sequenz, welche eine intramolekulare Helix III- und Helix 1-bildende Region ausbildet, einschließlich eines spaltbaren Motivs.

21. Zusammensetzung, enthaltend das asymmetrische Hammerhead-Ribozym nach einem der Ansprüche 8 bis 12, das RNA-Konstrukt nach Anspruch 13 oder 14, die DNA-Sequenz nach Anspruch 15, welche das asymmetrische Hammerhead-Ribozym oder das RNA-Konstrukt kodiert, oder den Vektor nach Anspruch 16, der das asymmetrische Hammerhead-Ribozym, das RNA-Konstrukt oder die DNA enthält, gegebenenfalls in Verbindung mit einem pharmazeutisch, veterinärmedizinisch oder landwirtschaftlich verträglichen Träger oder Verdünnungsmittel.

## Revendications

1. Séquence nucléotidique comprenant la formule générale suivante
3 ' - [N]₁₁ - [N]_{Lo} - [N]₁₀ - [N]₉₋₃ - [N] -5'
dans laquelle N représente des nucléotides quelconques pouvant être identiques ou différents, [N] ne représente aucun nucléotide, représente un nucléotide 2.1 ou des nucléotides 2.x à 2.1 aptes à former une hélice I dans un complexe cible / ribozyme en tête de marteau, [N]₉₋₃ représente des nucléotides 3 à 9 d'un ribozyme en tête de marteau, [N]₁₀ représente des nucléotides 10.1 à 10.n et [N]₁₁ ne représente aucun nucléotide, le nucléotide 11.m ou comprend au moins une parties des nucléotides 11.m à 11.1, x est un entier compris entre 2 et 8, m est un entier, n est un entier et représente la longueur de l'hélice II, dans laquelle, si [N]₁₁ comprend au moins une partie des nucléotides 11.m à 11.1, n et m peuvent être identiques ou différents , Lo est un entier et [N]_{Lo} représente une séquence quelconque apte à former une boucle 2 d'un ribozyme en tête de marteau, où ladite séquence nucléotidique ne contient pas la région formant l'hélice III d'un complexe cible / ribozyme en tête de marteau.

2. Séquence nucléotidique selon la revendication 1, dans laquelle la région 3' - [N]₁₁ - [N]_{Lo} - [N]₁₀ -5' contient au moins une séquence de reconnaissance pour une ou plusieurs enzymes de restriction et / ou une séquence de reconnaissance pour une enzyme de restriction qui chevauche la séquence suivante 3' à [N]₁₁.

3. Séquence nucléotidique selon la revendication 1 ou 2, dans laquelle n est compris entre 3 et 50.

4. Séquence nucléotidique selon l'une quelconque des revendications 1 à 3, dans laquelle x est 3 et / ou n est 4 et / ou m est compris entre 2 et 4.

5. Acide nucléique comprenant la séquence nucléotidique selon l'une quelconque des revendications 1 à 4 et une seconde séquence nucléotidique contenant au moins les nucléotides 12 à 14 d'un ribozyme en tête de marteau et au moins une partie de la région formant l'hélice III d'un complexe cible / ribozyme en tête de marteau, du nucléotide 15.1 au nucléotide 15.y, où y est un entier et le nombre de nucléotides dans la partie formant l'hélice I, correspondant aux nucléotides 2.1 à 2.x, est inférieur au nombre de nucléotides de la région formant l'hélice III, correspondant aux nucléotides 15.1 à 15.y.

6. Acide nucléique selon la revendication 5, dans lequel la séquence nucléotidique et la seconde séquence nucléotidique sont liées via une des séquences de reconnaissance contenues dans 3' - [N]₁₁ - [N]_{Lo} - [N]₁₀ -5' ou via une séquence de reconnaissance pour une enzyme de restriction qui chevauche la séquence suivante 3' à [N]₁₁.

7. Acide nucléique selon la revendication 5 ou 6 comprenant en outre une troisième séquence nucléotidique adjacente à la région formant l'hélice III de la deuxième séquence nucléotidique, contenant au moins 3 nucléotides aptes à former une hélice III intramoléculaire d'un ribozyme en tête de marteau, suivis directement du nucléotide 17 d'un ribozyme en tête de marteau et d'une séquence nucléotidique qui est complémentaire de [N].

8. Ribozyme asymétrique en tête de marteau comprenant l'acide nucléique selon l'une quelconque des revendications 5 à 7 ou la séquence nucléotidique selon l'une quelconque des revendications 1 à 4.

9. Ribozyme asymétrique en tête de marteau selon la revendication 8, dans lequel x est compris entre 2 et 8.

10. Ribozyme asymétrique en tête de marteau, dans lequel la région formant l'hélice I est plus petite que la région formant l'hélice III.

11. Ribozyme asymétrique en tête de marteau selon l'une quelconque des revendications 8 à 10, dans lequel au moins un nucléotide est modifié par voie chimique.

12. Ribozyme asymétrique en tête de marteau selon l'une quelconque des revendications 8 à 11, qui est obtenu par synthèse chimique.

13. Produit de synthèse d'ARN comprenant au moins deux ribozymes asymétriques en tête de marteau selon l'une quelconque des revendications 8 à 12.

14. Produit de synthèse d'ARN selon la revendication 13, dans lequel les régions formant l'hélice I et l'hélice III des ribozymes asymétriques en tête de marteau peuvent être identiques ou différentes.

15. Séquence d'ADN qui, lors d'une transcription, correspond au ribozyme asymétrique en tête de marteau selon l'une quelconque des revendications 8 à 12 ou au produit de synthèse d'ARN selon la revendication 13 ou 14.

16. Vecteur comprenant la séquence nucléotidique selon l'une quelconque des revendications 1 à 4, l'acide nucléique selon l'une quelconque des revendications 5 à 7, le ribozyme asymétrique en tête de marteau selon l'une quelconque des revendications 8 à 12, le produit de synthèse d'ARN de la revendication 13 ou 14 ou la séquence d'ADN de la revendication 15.

17. Organisme hôte contenant la séquence nucléotidique selon l'une quelconque des revendications 1 à 4, l'acide nucléique selon l'une quelconque des revendications 5 à 7, le ribozyme asymétrique en tête de marteau selon l'une quelconque des revendications 8 à 12, le produit de synthèse d'ARN de la revendication 13 ou 14, la séquence d'ADN de la revendication 15 ou le vecteur de la revendication 16.

18. Procédé de production de la séquence nucléotidique selon l'une quelconque des revendications 1 à 4, l'acide nucléique selon l'une quelconque des revendications 5 à 7, le ribozyme asymétrique en tête de marteau selon l'une quelconque des revendications 8 à 12, le produit de synthèse d'ARN de la revendication 13 ou 14, la séquence d'ADN de la revendication 15 ou le vecteur de la revendication 16, comprenant la culture de l'organisme hôte de la revendication 17 dans des conditions appropriées et l'isolation du produit souhaité de la culture.

19. Procédé de préparation du ribozyme asymétrique en tête de marteau selon l'une quelconque des revendications 8 à 12, du produit de synthèse d'ARN de la revendication 13 ou 14, ou de l'ADN codant ledit ribozyme asymétrique en tête de marteau ou de ledit produit de synthèse d'ARN, comprenant
(i) la sélection d'un motif sécable dans l'ARN cible,
(ii) la sélection du vecteur approprié et / ou de la cassette d'ADN qui fournit une boîte d'hélice I s'adaptant au motif sécable sélectionné et une partie du domaine catalytique d'un ribozyme en tête de marteau comme défini par la formule générale 3'-[N]₁₁ - [N]_{Lo} - [N]₁₀ - [N]₉₋₃ - [N] -5' et
(iii) la fixation de la deuxième séquence qui correspond à la partie formant l'hélice III et la partie résiduelle du domaine catalytique d'un ribozyme en tête de marteau.

20. Procédé de préparation du ribozyme asymétrique en tête de marteau selon l'une quelconque des revendications 8 à 12, du produit de synthèse d'ARN de la revendication 13 ou 14, ou de l'ADN codant ledit ribozyme asymétrique en tête de marteau ou dudit produit de synthèse d'ARN, comprenant l'introduction du domaine catalytique et de la séquence formant l'hélice I dans l'ADN par une seule étape de réaction de polymérisation en chaîne (PCR), dans laquelle un oligonucléotide d'ADN fournit la séquence d'au moins une partie du domaine catalytique et de la région formant l'hélice I, ou l'étape de la synthèse chimique d'au moins une partie des ribozymes asymétriques, ou la génération de ribozymes multiples en ajoutant une courte séquence signifiante constituant une région intramoléculaire formant l'hélice III et l'hélice I, incluant un motif sécable.

21. Composition contenant le ribozyme asymétrique en tête de marteau selon l'une quelconque des revendications 8 à 12, le produit de synthèse d'ARN de la revendication 13 ou 14, la séquence d'ADN de la revendication 15 codant ledit ribozyme asymétrique en tête de marteau ou ledit produit de synthèse d'ARN ou le vecteur de la revendication 16 contenant ledit ribozyme asymétrique en tête de marteau, ledit produit de synthèse d'ARN ou ladite séquence d'ADN, éventuellement en association avec un diluant et / ou un porteur acceptable du point pharmaceutique, vétérinaire ou agricole.
